# EUROPEAN PATENT SPECIFICATION

(11) **EP 0 656 012 B1**
(45) Date of publication and mention of the grant of the patent: **24.11.2004**
(21) Application number: 93917452.0
(22) Date of filing: 13.08.1993
(51) Int. Cl.: C07K 14/415, C12N 15/29, C12P 21/02, C12P 21/08, G01N 33/68, A61K 39/36, A61K 38/10

(54) **T CELL EPITOPES OF RYEGRASS POLLEN ALLERGEN**
T-ZELL EPITOPE DES RAYGRASPOLLEN-ALLERGENS
EPITOPES DE LYMPHOCYTES T DE l'ALLERGENE DE POLLEN DE L'IVRAIE VIVACE

(30) Priority: 14.08.1992 US 930060
(43) Date of publication of application: 07.06.1995
(73) Proprietor: THE UNIVERSITY OF MELBOURNE, Parkville, Victoria 3052 (AU)
(72) Inventor: KNOX, Robert, Bruce, North Balwyn, VIC 3104 (AU); SINGH, Mohan, Bir, Templestowe, VIC 3106 (AU); ROLLAND, Jennifer, Toorak, VIC 3142 (AU); BLAHER, Bella, North Caulfield, VIC 3161 (AU); SUPHIOGLU, Cenk, Keilor Downs, VIC 3038 (AU); MCCLUSKEY,James, South Australia 5061 (AU)
(74) Representative: Bizley, Richard Edward
(86) International application number: PCT/AU1993/000415
(87) International publication number: WO 1994/004564

(56) References cited:
- WO-A-93/04174
- AU-A- 2 824 492
- AU-A- 3 164 489
- AU-A- 8 408 391
- CLINICAL AND EXPERIMENTAL ALLERGY, vol. 22, no. 4, April 1992, pages 491-497, XP000644340 S.KLYSNER ET AL.: "Group V allergens in grass pollens:IV. Similarities in amino acid compositions and NH2-terminal sequences of the group V allergens from Lollium perenne, Poa pratensis and Dactylis glomerata"

### SEQUENCE LISTING

(1) GENERAL INFORMATION:
   (i) APPLICANT:
      (A) NAME: THE UNIVERSITY OF MELBOURNE
      (B) STREET: Grattan Street
      (C) CITY: Parkville
      (D) STATE: Victoria
      (E) COUNTRY: AUSTRALIA
      (F) POSTAL CODE (ZIP): 3052
      (G) TELEPHONE: 3-613-344-4000
      (H) TELEFAX: 3-613-344-7628
   (ii) TITLE OF INVENTION: T CELL EPITOPES OF RYEGRASS POLLEN ALLERGEN
   (iii) NUMBER OF SEQUENCES: 36
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: ASCII Text
   (v) CURRENT APPLICATION DATA:
      (A) APPLICATION NUMBER: 93917452.0 (PCT/AU93/00415)
      (B) FILING DATE: 13-AUG-1993
   (vi) PRIOR APPLICATION DATA:
      (A) APPLICATION NUMBER: US07/930060
      (B) FILING DATE: 14-AUG-1992
   (viii) ATTORNEY/AGENT INFORMATION:
      (A) NAME: Richard E. Bizley
         Hepworth Lawrence Bryer & Bizley
         Merlin House
         Falconry Court
         Bakers Lane
         Epping
         Essex
         CM16 5DQ
         UNITED KINGDOM
      (B) REGISTRATION NUMBER:
      (C) REFERENCE/DOCKET NUMBER: APEP94993
   (ix) TELECOMMUNICATION INFORMATION:
      (A) TELEPHONE: +44 (0)1992 561756
      (B) TELEFAX: +44 (0)1992 561934
(2) INFORMATION FOR SEQ ID NO:1:
   (i) SEQUENCE CHARACTERISTICS:
      (A) LENGTH: 1376 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 42..942
   (ix) FEATURE:
      (A) NAME/KEY: mat_peptide
      (B) LOCATION: 115..942
   (xi) SEQUENCE DESCRIPTION: SEQ ID NO:1:
   CGCTATCCCT CCCTCGTACA AACAAACGCA AGAGCAGCA ATG GCC GTC CAG AAG         54
                                             Met Ala Val Gln Lys
                                             -25
   TAC ACG GTG GCT CTA TTC CTC GCC GTG GCC CTC GTG GCG GGC CCG GCC       102
   Tyr Thr Val Ala Leu Phe Leu Ala Val Ala Leu Val Ala Gly Pro Ala
   -20                 -15                 -10                  -5
   GCC TCC TAC GCC GCT GAC GCC GGC TAC ACC CCC GCA GCC GCG GCC ACC       150
   Ala Ser Tyr Ala Ala Asp Ala Gly Tyr Thr Pro Ala Ala Ala Ala Thr
                         1               5                  10
   CCG GCT ACT CCT GCT GCC ACC CCG GCT GCG GCT GGA GGG AAG GCG ACG       198
   Pro Ala Thr Pro Ala Ala Thr Pro Ala Ala Ala Gly Gly Lys Ala Thr
            15                  20                  25
   ACC GAC GAG CAG AAG CTG CTG GAG GAC GTC AAC GCT GGC TTC AAG GCA       246
   Thr Asp Glu Gln Lys Leu Leu Glu Asp Val Asn Ala Gly Phe Lys Ala
        30                  35                  40
   GCC GTG GCC GCC GCT GCC AAC GCC CCT CCG GCG GAC AAG TTC AAG ATC       294
   Ala Val Ala Ala Ala Ala Asn Ala Pro Pro Ala Asp Lys Phe Lys Ile
    45                  50                  55                  60
   TTC GAG GCC GCC TTC TCC GAG TCC TCC AAG GGC CTC CTC GCC ACC TCC       342
   Phe Glu Ala Ala Phe Ser Glu Ser Ser Lys Gly Leu Leu Ala Thr Ser
                       65                  70                  75
   GCC GCC AAG GCA CCC GGC CTC ATC CCC AAG CTC GAC ACC GCC TAC GAC       390
   Ala Ala Lys Ala Pro Gly Leu Ile Pro Lys Leu Asp Thr Ala Tyr Asp
                   80                  85                  90
   GTC GCC TAC AAG GCC GCC GAG GGC GCC ACC CCC GAG GCC AAG TAC GAC       438
   Val Ala Tyr Lys Ala Ala Glu Gly Ala Thr Pro Glu Ala Lys Tyr Asp
           95                  100                 105
   GCC TTC GTC ACT GCC CTC ACC GAA GCG CTC CGC GTC ATC GCC GGC GCC      486
   Ala Phe Val Thr Ala Leu Thr Glu Ala Leu Arg Val Ile Ala Gly Ala
       110                 115                 120
   CTC GAG GTC CAC GCC GTC AAG CCC GCC ACC GAG GAG GTC CCT GCT GCT      534
   Leu Glu Val His Ala Val Lys Pro Ala Thr Glu Glu Val Pro Ala Ala
   125                 130                 135                 140
   AAG ATC CCC ACC GGT GAG CTG CAG ATC GTT GAC AAG ATC GAT GCT GCC      582
   Lys Ile Pro Thr Gly Glu Leu Gln Ile Val Asp Lys Ile Asp Ala Ala
                   145                 150                 155
   TTC AAG ATC GCA GCC ACC GCC GCC AAC GCC GCC CCC ACC AAC GAT AAG      630
   Phe Lys Ile Ala Ala Thr Ala Ala Asn Ala Ala Pro Thr Asn Asp Lys
               160                 165                 170
   TTC ACC GTC TTC GAG AGT GCC TTC AAC AAG GCC CTC AAT GAG TGC ACG      678
   Phe Thr Val Phe Glu Ser Ala Phe Asn Lys Ala Leu Asn Glu Cys Thr
           175                 180                 185
   GGC GGC GCC TAT GAG ACC TAC AAG TTC ATC CCC TCC CTC GAG GCC GCG      726
   Gly Gly Ala Tyr Glu Thr Tyr Lys Phe Ile Pro Ser Leu Glu Ala Ala
       190                 195                 200
   GTC AAG CAG GCC TAC GCC GCC ACC GTC GCC GCC GCG CCC GAG GTC AAG      774
   Val Lys Gln Ala Tyr Ala Ala Thr Val Ala Ala Ala Pro Glu Val Lys
   205                 210                 215                 220
   TAC GCC GTC TTT GAG GCC GCG CTG ACC AAG GCC ATC ACC GCC ATG ACC      822
   Tyr Ala Val Phe Glu Ala Ala Leu Thr Lys Ala Ile Thr Ala Met Thr
                   225                 230                 235
   CAG GCA CAG AAG GCC GGC AAA CCC GCT GCC GCC GCT GCC ACA GGC GCC      870
   Gln Ala Gln Lys Ala Gly Lys Pro Ala Ala Ala Ala Ala Thr Gly Ala
               240                 245                 250
   GCA ACC GTT GCC ACC GGC GCC GCA ACC GCC GCC GCC GGT GCT GCC ACC      918
   Ala Thr Val Ala Thr Gly Ala Ala Thr Ala Ala Ala Gly Ala Ala Thr
           255                 260                 265
   GCC GCT GCT GGT GGC TAC AAA GCC TGATCAGCTT GCTAATATAC TACTGAACGT     972
   Ala Ala Ala Gly Gly Tyr Lys Ala
       270                 275
   ATGTATGTGC ATGATCCGGG CGGCGAGTGG TTTTGTTGAT AATTAATCTT CGTTTTCGTT   1032
   TCATGCAGCC GCGATCGAGA GGGCTTGCAT GCTTGTAATA ATTCAATATT TTTCATTTCT   1092
   TTTTGAATCT GTAAATCCCC ATGACAAGTA GTGGGATCAA GTCGGCATGT ATCACCGTTG   1152
   ATGCGAGTTT AACGATGGGG AGTTTATCAA AGAATTTATT ATTAAAAAAA AAAAAAAAAA   1212
   AAAAAAAAAA AAAAAAAGAG CACGCTATCC CTCCCTCGTA CAAACAAACG CAAGAGCAGC   1272
   AATGGCCGTC CAGAAGTACA CGGTGGCTCT ATTCCTCGCC GTGGCCCTCG TGGCGGGCCC 1332
   GGCCGCCTCC TACGCCGCTG ACGCTTAAAC ATTCATATCA TCAT 1376
(2) INFORMATION FOR SEQ ID NO:2:
   (i) SEQUENCE CHARACTERISTICS:
      (A) LENGTH: 301 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Ala Val Gln Lys Tyr Thr Val Ala Leu Phe Leu Ala Val Ala Leu
   -25                 -20                 -15                 -10
   Val Ala Gly Pro Ala Ala Ser Tyr Ala Ala Asp Ala Gly Tyr Thr Pro
                    -5                   1               5
   Ala Ala Ala Ala Thr Pro Ala Thr Pro Ala Ala Thr Pro Ala Ala Ala
            10                  15                  20
   Gly Gly Lys Ala Thr Thr Asp Glu Gln Lys Leu Leu Glu Asp Val Asn
        25                  30                  35
   Ala Gly Phe Lys Ala Ala Val Ala Ala Ala Ala Asn Ala Pro Pro Ala
    40                  45                  50                  55
   Asp Lys Phe Lys Ile Phe Glu Ala Ala Phe Ser Glu Ser Ser Lys Gly
                    60                  65                  70
   Leu Leu Ala Thr Ser Ala Ala Lys Ala Pro Gly Leu Ile Pro Lys Leu
                75                  80                  85
   Asp Thr Ala Tyr Asp Val Ala Tyr Lys Ala Ala Glu Gly Ala Thr Pro
            90                  95                 100
   Glu Ala Lys Tyr Asp Ala Phe Val Thr Ala Leu Thr Glu Ala Leu Arg
       105                 110                 115
   Val Ile Ala Gly Ala Leu Glu Val His Ala Val Lys Pro Ala Thr Glu
   120                 125                 130                 135
   Glu Val Pro Ala Ala Lys Ile Pro Thr Gly Glu Leu Gln Ile Val Asp
                   140                 145                 150
   Lys Ile Asp Ala Ala Phe Lys Ile Ala Ala Thr Ala Ala Asn Ala Ala
               155                 160                 165
   Pro Thr Asn Asp Lys Phe Thr Val Phe Glu Ser Ala Phe Asn Lys Ala
           170                 175                 180
   Leu Asn Glu Cys Thr Gly Gly Ala Tyr Glu Thr Tyr Lys Phe Ile Pro
       185                 190                 195
   Ser Leu Glu Ala Ala Val Lys Gln Ala Tyr Ala Ala Thr Val Ala Ala
   200                 205                 210                 215
   Ala Pro Glu Val Lys Tyr Ala Val Phe Glu Ala Ala Leu Thr Lys Ala
                   220                 225                 230
   Ile Thr Ala Met Thr Gln Ala Gln Lys Ala Gly Lys Pro Ala Ala Ala
               235                 240                 245
   Ala Ala Thr Gly Ala Ala Thr Val Ala Thr Gly Ala Ala Thr Ala Ala
           250                 255                 260
   Ala Gly Ala Ala Thr Ala Ala Ala Gly Gly Tyr Lys Ala
       265                 270                 275
(2) INFORMATION FOR SEQ ID NO:3:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:3:
   Ala Asp Ala Gly Tyr Thr Pro Ala Ala Ala Ala Thr
   1               5                   10
(2) INFORMATION FOR SEQ ID NO:4:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:4:
   Ala Ala Ala Thr Pro Ala Thr Pro Ala Ala Thr Pro Ala
   1               5                   10
(2) INFORMATION FOR SEQ ID NO:5:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:5:
   Ala Ala Thr Pro Ala Ala Ala Gly Gly Lys Ala Thr
   1               5                   10
(2) INFORMATION FOR SEQ ID NO:6:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:6:
   Gly Lys Ala Thr Thr Asp Glu Gln Lys Leu Leu Glu
   1               5                   10
(2) INFORMATION FOR SEQ ID NO:7:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:7:
   Lys Leu Leu Glu Asp Val Asn Ala Gly Phe Lys Ala
   1               5                   10
(2) INFORMATION FOR SEQ ID NO:8:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:8:
   Gly Phe Lys Ala Ala Val Ala Ala Ala Ala Asn Ala
   1               5                   10
(2) INFORMATION FOR SEQ ID NO:9:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:9:
   Ala Ala Asn Ala Pro Pro Ala Asp Lys Phe Lys Ile
   1               5                   10
(2) INFORMATION FOR SEQ ID NO:10:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:10:
   Lys Phe Lys Ile Phe Glu Ala Ala Phe Ser Glu Ser
   1               5                   10
(2) INFORMATION FOR SEQ ID NO:11:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:11:
   Phe Ser Glu Ser Ser Lys Gly Leu Leu Ala Thr Ser
   1               5                   10
(2) INFORMATION FOR SEQ ID NO:12:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:12:
   Leu Ala Thr Ser Ala Ala Lys Ala Pro Gly Leu Ile
   1               5                   10
(2) INFORMATION FOR SEQ ID NO:13:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:13:
   Pro Gly Leu Ile Pro Lys Leu Asn Thr Ala Tyr Asp
   1               5                   10
(2) INFORMATION FOR SEQ ID NO:14:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:14:
   Thr Ala Tyr Asp Val Ala Tyr Lys Ala Ala Glu Gly
   1               5                   10
(2) INFORMATION FOR SEQ ID NO:15:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:15:
   Ala Ala Glu Gly Ala Thr Pro Glu Ala Lys Tyr Asp
   1               5                   10
(2) INFORMATION FOR SEQ ID NO:16:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:16:
   Ala Lys Tyr Asp Ala Phe Val Thr Ala Leu Thr Glu
   1               5                   10
(2) INFORMATION FOR SEQ ID NO:17:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:17:
   Ala Leu Thr Glu Gly Leu Arg Val Ile Ala Gly Ala
   1               5                   10
(2) INFORMATION FOR SEQ ID NO:18:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:18:
   Ile Ala Gly Ala Leu Glu Val His Ala Val Lys Pro Ala
   1               5                   10
(2) INFORMATION FOR SEQ ID NO:19:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:19:
   Ala Val Lys Pro Ala Thr Glu Glu Val Pro Ala Ala
   1               5                   10
(2) INFORMATION FOR SEQ ID NO:20:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:20:
   Val Pro Ala Ala Lys Ile Pro Thr Gly Glu Leu Gln Ile
   1 5 10
(2) INFORMATION FOR SEQ ID NO:21:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:21:
   Gly Glu Leu Gln Ile Val Asp Lys Ile Asp Ala Ala
   1 5 10
(2) INFORMATION FOR SEQ ID NO:22:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:22:
   Ile Asp Ala Ala Phe Lys Ile Ala Ala Thr Ala Ala
   1 5 10
(2) INFORMATION FOR SEQ ID NO:23:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:23:
   Ala Thr Ala Ala Asn Ala Ala Pro Thr Asn Asp Lys
   1 5 10
(2) INFORMATION FOR SEQ ID NO:24:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:24:
   Thr Asn Asp Lys Phe Thr Val Phe Glu Ser Ala Phe
   1               5                   10
(2) INFORMATION FOR SEQ ID NO:25:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:25:
   Glu Ala Ser Phe Asn Lys Ala Leu Asn Glu Cys Thr
   1               5                   10
(2) INFORMATION FOR SEQ ID NO:26:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:26:
   Asn Glu Cys Thr Gly Gly Ala Tyr Glu Thr Tyr Lys
   1               5                   10
(2) INFORMATION FOR SEQ ID NO:27:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:27:
   Glu Thr Tyr Lys Phe Ile Pro Ser Leu Glu Ala Ala
   1               5                   10
(2) INFORMATION FOR SEQ ID NO:28:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:28:
   Leu Glu Ala Ala Val Lys Gln Ala Tyr Ala Ala Thr
   1               5                   10
(2) INFORMATION FOR SEQ ID NO:29:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:29:
   Tyr Ala Ala Thr Val Ala Ala Pro Glu Val Lys Tyr
   1               5                   10
(2) INFORMATION FOR SEQ ID NO:30:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:30:
   Glu Val Lys Tyr Ala Val Phe Glu Ala Ala Leu Thr
   1               5                   10
(2) INFORMATION FOR SEQ ID NO:31:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:31:
   Ala Ala Leu Thr Lys Ala Ile Thr Ala Met Thr Gln Ala
   1               5                   10
(2) INFORMATION FOR SEQ ID NO:32:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:32:
   Ala Met Thr Gln Ala Gln Lys Ala Gly Lys Pro Ala
   1               5                   10
(2) INFORMATION FOR SEQ ID NO:33:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:33:
   Gly Lys Pro Ala Ala Ala Ala Ala Thr Ala Ala Ala
   1               5                   10
(2) INFORMATION FOR SEQ ID NO:34:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:34:
   Thr Ala Ala Ala Thr Val Ala Thr Ala Ala Ala Thr
   1               5                   10
(2) INFORMATION FOR SEQ ID NO:35:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:35:
   Ala Ala Ala Thr Ala Ala Ala Gly Ala Ala Thr Ala
   1               5                   10
(2) INFORMATION FOR SEQ ID NO:36:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:36:
   Gly Ala Ala Thr Ala Ala Ala Gly Gly Tyr Lys Ala
   1               5                   10

## Description

Allergens constitute the most abundant proteins of grass pollen. which is the major cause of allergic disease in temperate climates (Marsh (1975) Allergens and the genetics of allergy: in M. Sela (ed). The Antigens, Vol. 3, pp 271-359, Academic Press Inc., London, New York)., Hill et al. (1979) Medical Journal of Australia 1, 426-429). The first descriptions of the allergenic proteins in ryegrass showed that they are immunochemically distinct. and are known as groups I, II, III and IV (Johnson and Marsh (1965) Nature, 206, 935-942; and Johnson and Marsh (1966) Immunochemistry 3, 91-100). Using the International Union of Immunological Societies' (IUIS) nomenclature, these allergens are designated *Lol p I*, *Lol p* II, *Lol p* III and *Lol p* IV. In addition, another important *Lolium prenne L*. allergen which has been identified in the literature is *Lol p V* also known as *Lol p IX* or *Lol p Ib* (Singh et al. (1991) *Proc. Natl. Acad. Sci. USA*. 88:1384-1388. Suphioglu, et al. 1992. Lancet. 339: 569-572.

These five proteins have been identified in pollen ryegrass, *Lolium perenne* L., and act as allergens in triggering immediate (Type 1) hypersensitivity in susceptible humans.

*Lol p V* is defined as an allergen because of its ability to bind to specific IgE in sera of ryegrass-sensitive patients. to act as an antigen in IgG responses and to trigger T-cell responses. The allergenic properties have been demonstrated by immunoblotting studies showing 80% of ryegrass pollen sensitive patients possessed specific IgE antibody that bound to *Lol p* V isoforms (PCT applicaton publication no. WO 93/04174, page 65). These results indicate that *Lol p V* is a major ryegrass allergen.

Substantial allergenic cross-reactivity between grass pollens has been demonstrated using an IgE-binding assay, the radioallergo-sorbent test (RAST), for example, as described by Marsh et al. (1970) J. Allergy, 46, 107-121. and Lowenstein (1978) Prog. Allergy. 25, 1-62. (Karger, Basel).

The immunochemical relationship of *Lol p* V with other grass pollen antigens have been demonstrated using both polyclonal and monoclonal antibodies (Zhang et al., *Int Arch Allergy Appl Immunol*, **96**:28-34 (1991); Roberts et al., *Int Arch Allergy Appl Immunol*, **98**:178-180 (1992); and Mattheisen and Lowenstein, *Clinical and Experimental Allergy*, **21**: 309-320 (1991). Antibodies have been prepared to purified proteins that bind IgE components. These data demonstrate that a major allergen is present in pollen of closely related grasses is immunochemically similar to *Lol p* V and are generally characterized as Group V allergens.

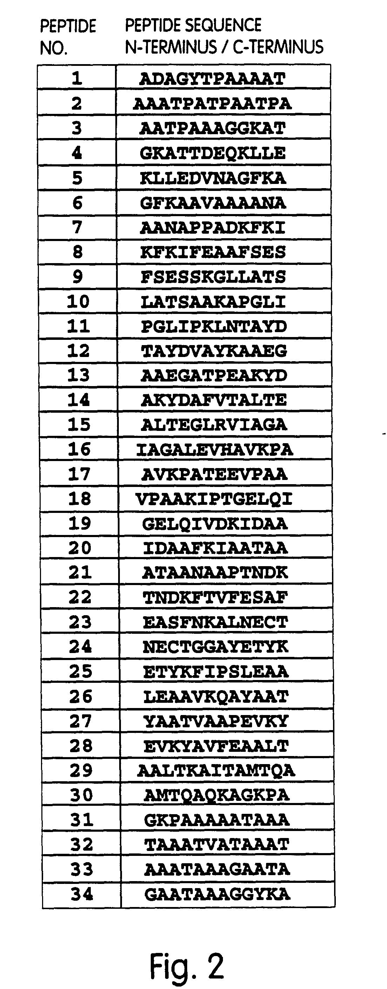

Accordingly, the present invention provides an isolated peptide fragment of *Lol p* V or a homologue thereof of no more than 45 amino acid residues and comprising an amino acid sequence selected from amino acid sequences as shown in Fig. 2 of peptides 1 (SEQ ID NO:3), 6 (SEQ ID NO:8), 7 (SEQ ID NO:9), 12 (SEQ ID NO:14), 14 (SEQ ID NO:16), 16 (SEQ ID NO:18), 19 (SEQ ID NO:21), 21 (SEQ ID NO:23), 22 (SEQ ID NO:24), 23 (SEQ ID NO:25), 24 (SEQ ID NO:26), 25 (SEQ ID NO:27), 28 (SEQ ID NO:30), 30 (SEQ ID NO:32), 33 (SEQ ID NO:35) and 34 (SEQ ID NO:36).

The isolated peptide fragments may either-
(a) comprise at least one T cell epitope of Lol p V, said peptides comprising an amino acid sequence selected from the amino acid sequences as shown in Fig. 2 of peptides 6 (SEQ ID NO:8), 7 (SEQ ID NO:9), 12 (SEQ ID NO:14), 14 (SEQ ID NO:16), 16 (SEQ ID NO:18), 19 (SEQ ID NO:21), 22 (SEQ ID NO:24), 25 (SEQ ID NO:27), 28 (SEQ ID NO:30), 33 (SEQ ID NO:35) and 34 (SEQ ID NO:36); or
(b) comprise at least one B cell epitope of Lol p V, said peptides comprising an amino acid sequence selected from the group consisting of amino acid sequences as shown in Fig. 2 of peptides 1 (SEQ ID NO:3), 7 (SEQ ID NO:9), 16 (SEQ ID NO:18), 19 (SEQ ID NO:21), 21 (SEQ ID NO:23), 23 (SEQ ID NO:25), 24 (SEQ ID NO:26), 28 (SEQ ID NO:30), 30(SEQ ID NO:32) and 34 (SEQ ID NO:36).

The isolated peptide fragments of the invention have a T cell stimulation index of at least 2.0.

In preferred embodiments the isolated peptide fragments, when administered to an individual sensitive to *Lol* p V allergen induce T cell anergy in the individual or modify the lymphokine secretion profile of T cells in the individual.

In other embodiments the isolated peptide fragments may either:-
(a) not bind immunoglobulin E specific for *Lol p* V in at least about 75% of individuals sensitive to *Lol p* V, or if binding of the peptide or portion thereof to said immunoglobulin E occurs, such binding does not result in release of mediators from mast cells or basophils in a substantial percentage of individuals sensitive to *Lol p* V; or
(b) bind immunoglobulin E to a lesser extent than purified native *Lol p* V binds immunoglobulin E.

The amino acid sequence of peptide fragments of the invention may be modified by amino acid substitution, addition or deletion, optionally to include one or more polymorphisms, and/or the fragment may be modified using the polyethylene glycol (PEG) method of Wie *et al* (1981) Int. Arch. Allergy Appl. Immunol. 64:84-99, and/or reduction/alkylation, acylation or mild formalin treatment.

Preferred modified peptide fragments have the ability to induce T-cell anergy and bind MHC proteins without the ability to induce a proliferative response on administration.

The invention also provides an isolated nucleic acid having a sequence encoding a peptide as hereinbefore described or a functional equivalent of said nucleic acid sequence

Also provided by the invention are isolated peptides produced in host cells transformed with a nucleic acid as hereinbefore described.

The invention includes expression vectors comprising nucleic acids are hereinbefore described.

The invention also provides a therapeutic composition comprising at least one isolated peptide as hereinbefore described and a pharmaceutically acceptable carrier or diluent.

Preferred compositions of the invention comprises a combination of peptides selected from:
14 (SEQ ID NO:16) and 22 (SEQ ID NO:24);
19 (SEQ ID NO:21) and 22 (SEQ ID NO:24);
19 (SEQ ID NO:21) and 25 (SEQ ID NO:27);
14 (SEQ ID NO:16), 22 (SEQ ID NO:24), and 25 (SEQ ID NO:27);
19 (SEQ ID NO:21), 22 (SEQ ID NO:24), and 25 (SEQ ID NO:27);
14 (SEQ ID NO:16), 22 (SEQ ID NO:24), 25 (SEQ ID NO:27, and 34 (SEQ ID NO:36);
19 (SEQ ID NO:21), 22 (SEQ ID NO:24), 25 (SEQ ID NO:27), and 34 (SEQ ID NO:36);
14 (SEQ ID NO:16), 19 (SEQ ID NO:21), 22 (SEQ ID NO:24), and 25 (SEQ ID NO:27);
14 (SEQ ID NO:16), 19 (SEQ ID NO:21), 22 (SEQ ID NO:24), 25 (SEQ ID NO:27), and 34 (SEQ ID NO:36);
6 (SEQ ID NO:8), 7 (SEQ ID NO:9), 9 (SEQ ID NO:11), 12 (SEQ ID NO:14),
14 (SEQ ID NO:16), 16 (SEQ ID NO:18), 19 (SEQ ID NO:21), 22 (SEQ ID NO:24), 25 (SEQ ID NO:27), 33 (SEQ ID NO:35), and 34 (SEQ ID NO:36);
6 (SEQ ID NO:8), 9 (SEQ ID NO:11), 16 (SEQ ID NO:18), 19 (SEQ ID NO:21), 22 (SEQ ID NO:24), and 34 (SEQ ID NO:36); and
6(SEQ ID NO:8), 9 (SEQ ID NO:11), 14 (SEQ ID NO:16), 16 (SEQ ID NO:18),
19 (SEQ ID NO:21), 22 (SEQ ID NO:24), 25 (SEQ ID NO:27), and 34 (SEQ ID NO:36).

The invention includes the use of a composition as hereinbefore described for the manufacture of a medicament for treating sensitivity to *Lol p* V allergen or an allergen which is immunologically cross reactive with *Lol p* V allergen.

The invention also includes the use of at least two compositions as hereinbefore described for the manufacture of a medicament for treating sensitivity to *Lol p* V allergen which is immunologically cross reactive with ryegrass pollen allergen in an individual.

The invention further includes the use of a peptide as hereinbefore described for the manufacture of a medicament for treating sensitivity to *Lol p* V allergen or an allergen which is immunologically cross reactive with *Lol p* V allergen.

The invention also provides products containing at least two peptides as hereinbefore described and/or compositions as hereinbefore described as a combined preparation for simultaneous, separate or sequential use in treating sensitivity to *Lol p* V allergen or an allergen which is cross reactive with ryegrass pollen allergen.

In another aspect the invention provides a method of detecting sensitivity to *Lol p* V in an individual, comprising combining a blood sample obtained from the individual with at least one peptide as hereinbefore described under conditions appropriate for binding of blood components with the peptide, and determining the extent to which such binding occurs as indicative of sensitivity in the individual to ryegrass pollen; optionally the extent to which binding occurs being determined by assessing B cell function, T cell function, T cell proliferation or a combination of T cell function and B cell proliferation.

A particularly preferred embodiment of the invention is provided by a therapeutic composition comprising a pharmaceutically acceptable carrier or diluent and at least two *Lol p* V peptides, selected from the group consisting of peptides, 6 (SEQ ID NO:8), 7 (SEQ ID NO:9), 9 (SEQ ID NO:11), 12 (SEQ ID NO:14), 16 (SEQ ID NO:18), 19 (SEQ ID NO:21), 22 (SEQ ID NO:24), 28 (SEQ ID NO:30) and 34 (SEQ ID NO:36). Suitably said composition comprises a sufficient percentage of the T cell epitopes of *Lol p* V such that upon administration of the composition to an individual sensitive to *Lol p* V, T cells of the individual are tolerized to *Lol p* V.

Peptides to be used therapeutically within the scope of the invention comprise at least one T cell epitope, preferably at least two T cell epitopes of *Lol p* V. The invention further provides peptides comprising at least two regions, each region comprising at least one T cell epitope of *Lol p* V. Peptides of the invention to be used for diagnostic purposes are capable of binding IgE and comprise at least one B cell epitope.

Modified peptides of the invention have similar or enhanced therapeutic properties as the corresponding, naturally-occurring allergen or portion thereof, but have reduced side effects. Modified peptides may have improved properties such as increased solubility and stability. Therapeutic peptides of the invention are capable of modifying, in a *Lol p* V-sensitive individual to whom they are administered, the allergic response of the individual to *Lol p* V or an allergen immunologically cross-reactive *Lol p* V.

Further features of the present invention will be better understood from the following detailed description of the preferred embodiments of the invention in conjunction with the appended figures.

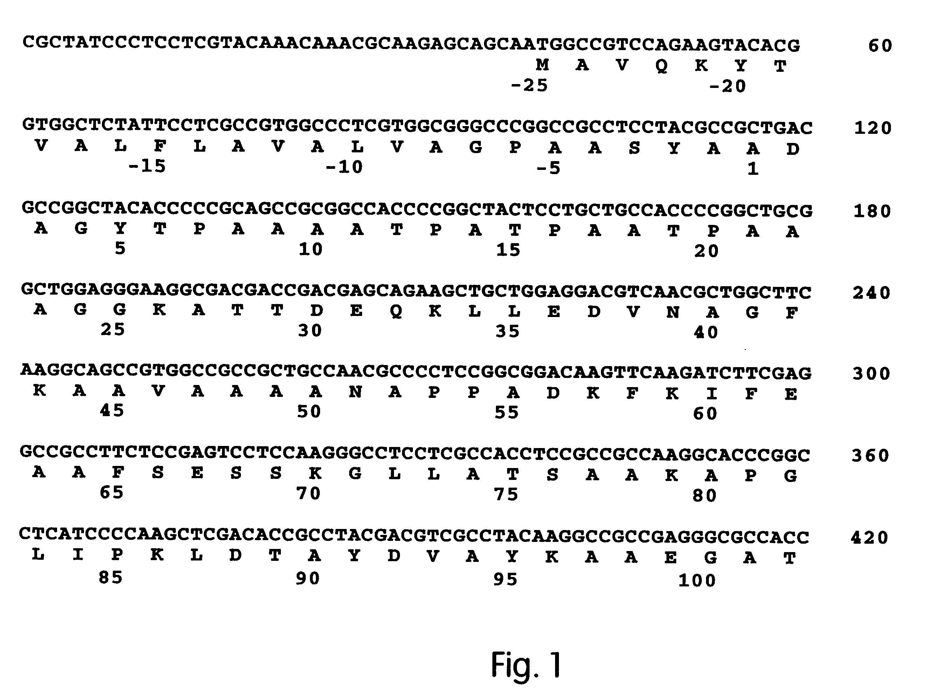

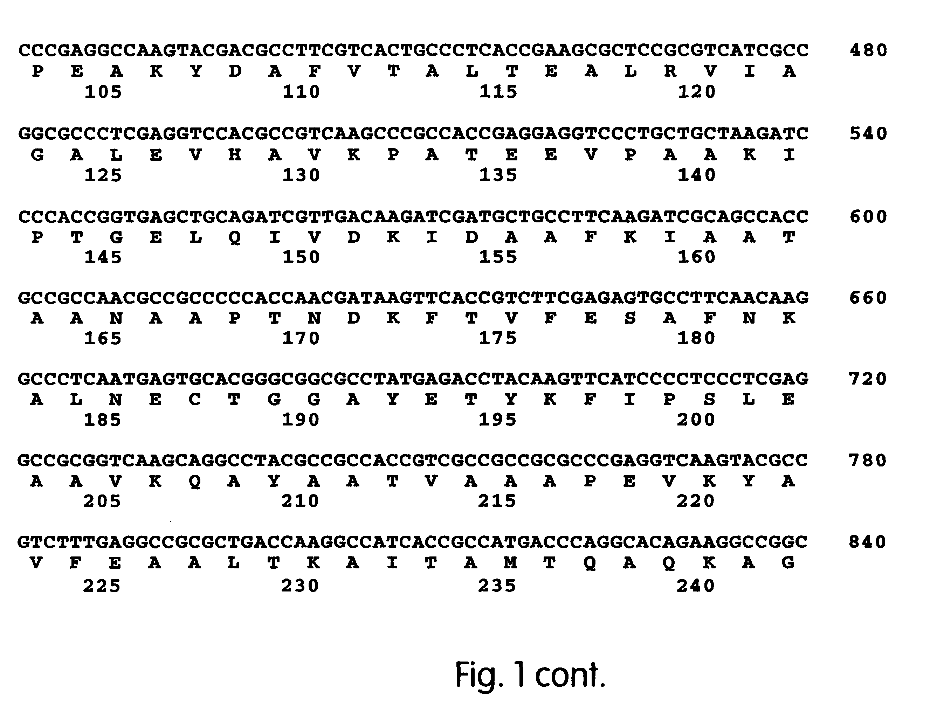

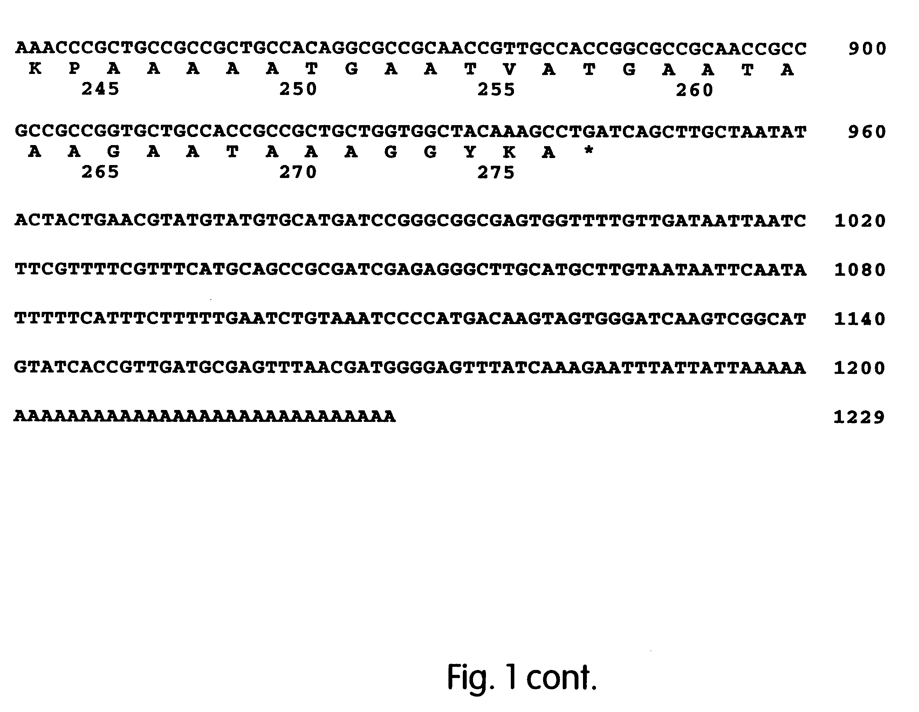

Fig. 1 shows the nucleotide sequence of cDNA clone 12R (SEQ ID NO:1) and its predicted amino acid sequence (SEQ ID NO:2). Clone 12R is a full-length clone of *Lol p* V.

Fig. 2 shows various peptides of the invention of various lengths derived from *Lol p* V.

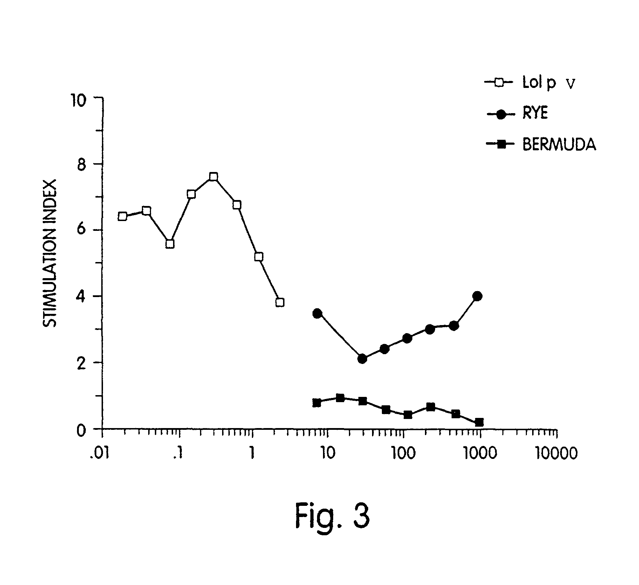

Fig. 3 is a graphic representation of the stimulation index of T cells from day 34 of the T cell line cultured with autologous irradiated PBMC in the presence of the inducing allergen *Lol p* V, crude ryegrass pollen extract or crude Bermuda grass pollen extract; proliferation as assessed by [3H]TdR incorporation, the results are expressed as the stimulation index, determined by dividing the average CPM of cells with allergen by the average CPM of cells with medium, alone, each allergen concentration was tested in triplicate.

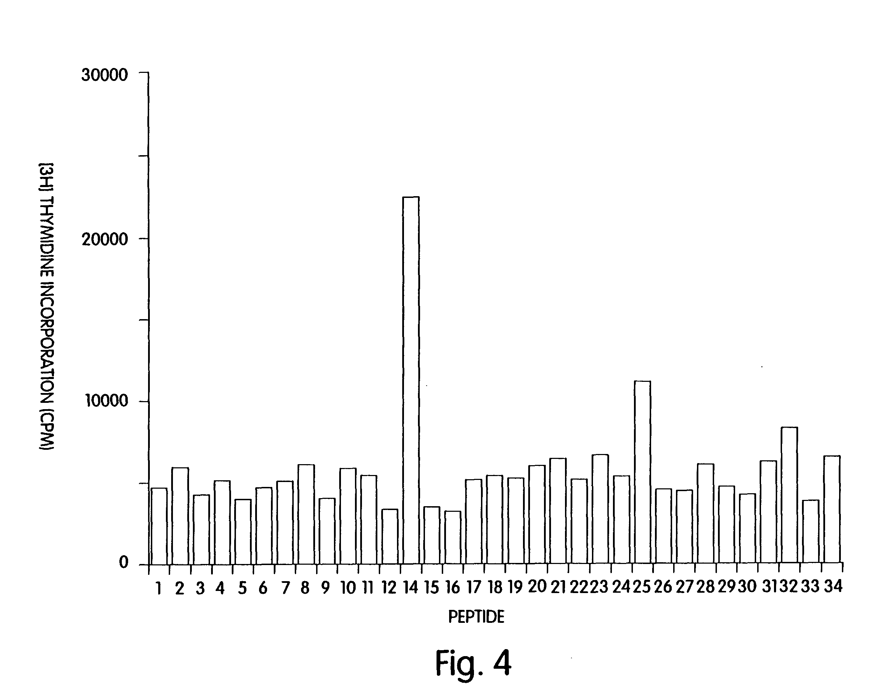

Fig. 4 is graphic representation of the proliferative response of a T cell line derived from a healthy ryegrass pollen allergic adult, on day 49 to the *Lol p* V synthetic peptides, the results are expressed as the average counts per minute (CPM) of duplicate cultures, background response of cells in the absence of allergen was 4849 CPM.

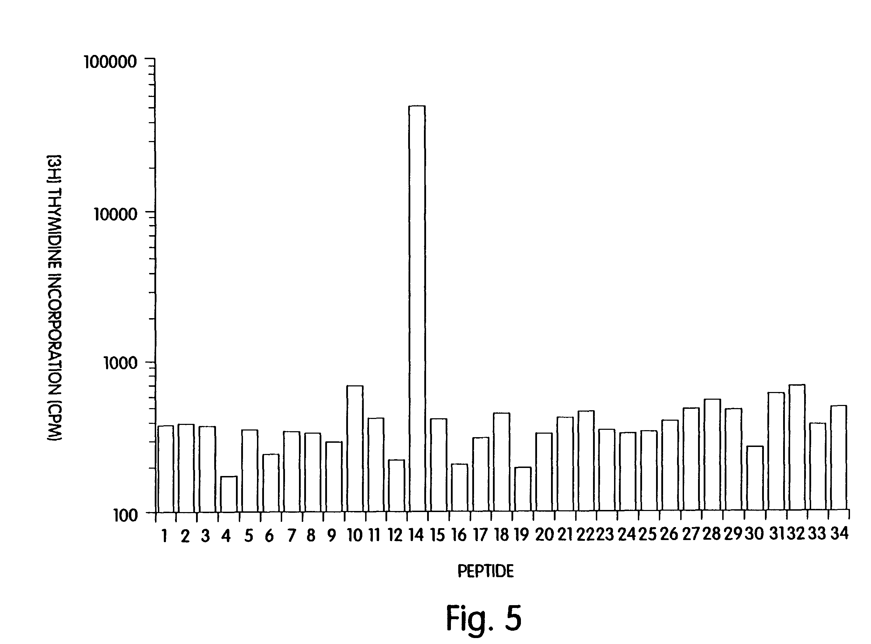

Fig. 5 is a graphic representation of the proliferative response of T cell clone A12 to the *Lol p* V synthetic peptides, the results are expressed as the average counts per minute (CPM) of duplicate cultures, background response of cells in the absence of allerge was 358 CPM, and the Y-axis is shown on a log scale.

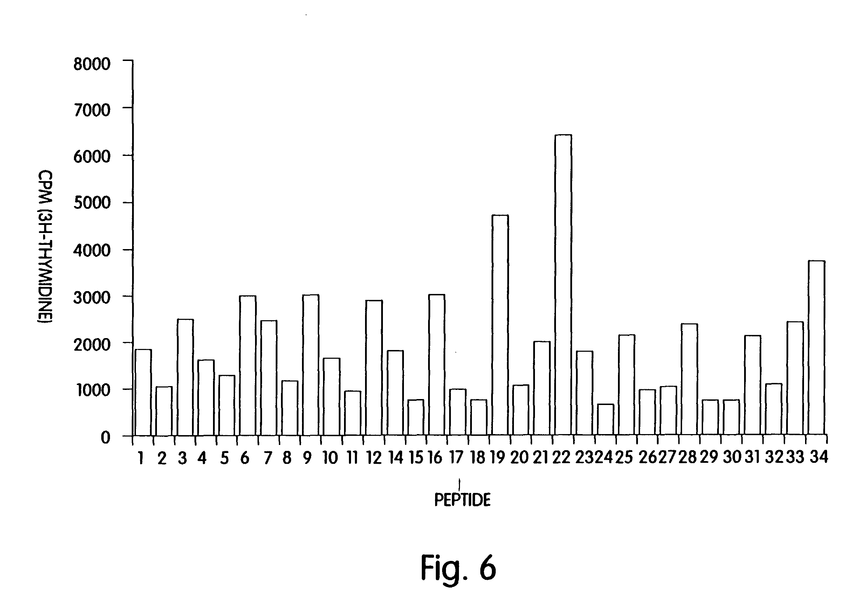

Fig. 6 is a graphic representation of the proliferative response of a second T cell line derived from a healthy ryegrass pollen allergic adult, to the *Lol p* V synthetic peptides, the results are expressed as the average counts per minute (CPM), the background response of cells in the absence of allergen was 2500 CPM, and the concentration of peptide was 0.1 µg/ml.

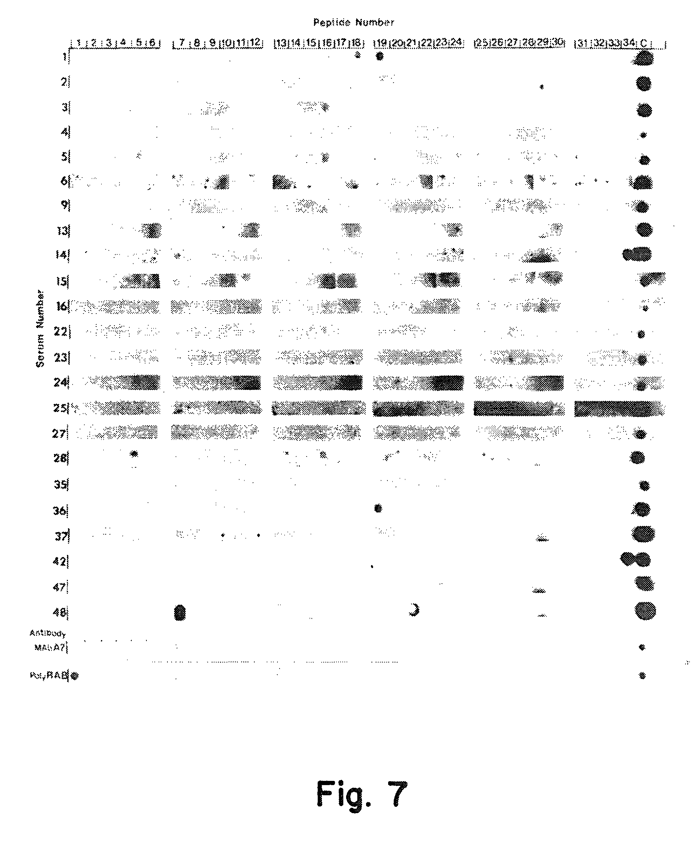

Fig. 7 shows a dot blot immunoassay of the 35 overlapping peptides from *Lol p* V, (clone 12R) immobilized on nitrocellulose (NC) filter and screened with individual sera showing human IgE binding. mAbs FMC-A7 and polyclonal anti-*Lol p* V rabbit antibody, C represents crude ryegrass pollen extract (1 µg/dot) used as a positive control.

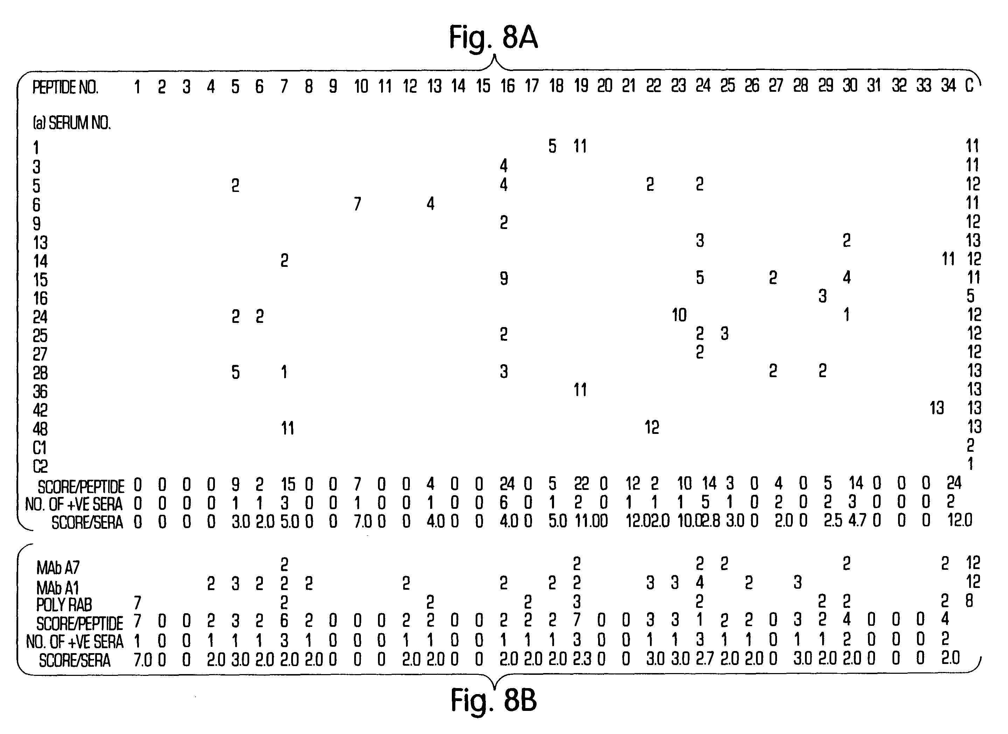

Fig. 8 shows identification of B cell epitopes of *Lol p* V based on dot immonoassays of overlapping sequential synthetic peptides derived from clone 12R; Fig. 8a shows the IgE binding from 16 positive sera from a total of 50 ryegrass pollen allergeic patients (Rast≥4); and Fig. 8b shows monoclonal or polyclonal antibody binding, the values are arbitrary densitometric units of the intensity of the dot blot, measured by Pharmacia LKB UltraScan XL. Sweden. densometric values ≥2 were considered as positive binding in comparison to the background. for every peptide. the sera and antibody values have been added (score/peptide) and divided by the number of positive sera or antibody to express the final value as an average (score/sera or antibody). sera Cl and C2 were used as negative controls (C refers to crude ryegrass pollen extract (1 µg/dot) as a positive control).

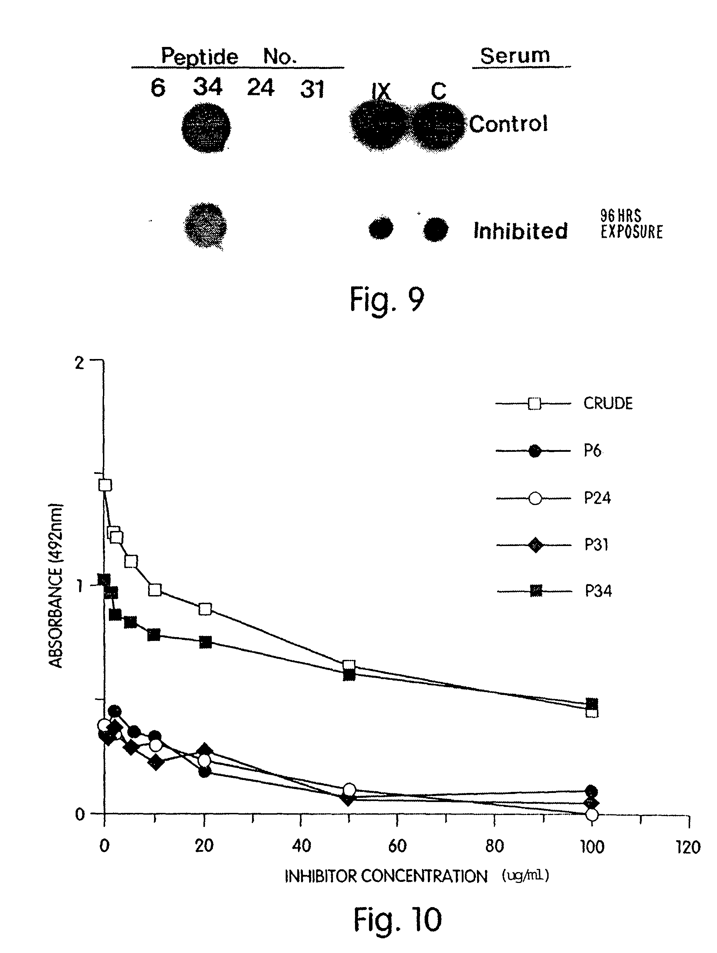

Fig. 9 shows an inhibition peptide dot-blot with peptide 34 (SEQ ID NO:36), peptides 6 (SEQ ID NO:8), 24 (SEQ ID NO:26), and 31 (SEQ ID NO:33) were used as negative controls; FPLC purified Lol p V designated as "IX" and crude ryegrass pollen extract designated as "C" were used as positive controls.

Fig. 10 is a graphic depiction of an Inhibition ELISA with peptide 34 (SEQ ID NO:36), peptides 6 (SEQ ID NO:8), 24 (SEQ ID NO:26), and 31 (SEQ ID NO:33) were used as negative controls: and crude ryegrass pollen extract was used as a positive control.

As used herein, a peptide or fragment of a protein refers to an amino acid sequence having fewer amino acid residues than the entire amino acid sequence of the protein. The terms "isolated" and "purified" as used herein refer to peptides of the invention which are substantially free of cellular material or culture medium when produced by recombinant DNA techniques. or substantially free of chemical precursors or other chemicals when synthesized chemically. As used herein, the term "peptide" of the invention include peptides derived from *Lol p* V which comprise at least one T cell epitope of the allergen or a portion of such peptide which comprises at least one T cell epitope and/or peptides derived from *Lol p* V which binds IgE and which comprise at least one B cell epitope.

Peptides comprising at least two regions, each region comprising at least one T cell epitope of *Lol p* V are also within the scope of the invention. Isolated peptides or regions of isolated peptides, each comprising at least two T cell epitopes of *Lol p V* protein allergen are particularly desirable for increased therapeutic effectiveness. Peptides which are immunologically related (e.g., by antibody or T cell cross-reactivity) to peptides of the present invention are also within the scope of the invention. Peptides immunologically related by antibody cross-reactivity are bound by antibodies specific for a peptide of *Lol p* V Peptides immunologically related by T cell cross-reactivity are capable of reacting with the same T cells as a peptide of the invention.

Isolated peptides of the invention can be produced by recombinant DNA techniques in a host cell transformed with a nucleic acid having a sequence encoding such peptide. The isolated peptides of the invention can also be produced by chemical synthesis. When a peptide is produced by recombinant techniques. host cells transformed with a nucleic acid having a sequence encoding a peptide of the invention the functional equivalent of the nucleic acid sequence are cultured in a medium suitable for the cells and peptides can be purified from cell culture medium, host cells. or both using techniques known in the art for purifying peptides and proteins including ion-exchange chromatography, gel filtration chromatography, ultrafiltration. electrophoresis or immunopurification with antibodies specific for the peptide, the protein allergen from which the peptide is derived, or a portion thereof.

Nucleic acid coding for a *Lol p V* peptide of the invention or at least one fragment thereof may be expressed in bacterial cells such as *E*. *coli.* insect cells, yeast, or mammalian cells such as Chinese hamster ovary cells (CHO). Suitable expression vectors, promoters, enhancers, and other expression control elements may be found in Sambrook et al. *Molecular Cloning: A Laboratory* *Manual*, second edition, Cold Spring Harbor Laboratory Press. Cold Spring Harbor. New York. 1989. Other suitable expression vectors, promoters, enhancers, and other expression elements are known to those skilled in the art. Suitable vectors for expression in yeast include YepSec1 (Baldari et al. (1987) *Embo J*. 6: 229-234); pMFa (Kurjan and Herskowitz (1982) *Cell* 30: 933-943); JRY88 (Schultz et al. (1987) *Gene* 54: 113-123) and pYES2 (Invitrogen Corporation, San Diego, CA). These vectors are freely available. Baculovirus and mammalian expression systems are also available. For example, a baculovirus system is commercially available (PharMingen, San Diego, CA) for expression in insect cells while the pMSG vector is commercially available (Pharmacia, Piscataway. NJ) for expression in mammalian cells.

For expression in *E*. *coli*, suitable expression vectors include, among others, pTRC (Amann et al. (1988) *Gene* 69: 301-315); pGEX (Amrad Corp., Melbourne. Australia); pMAL (N.E. Biolabs, Beverly. MA); pRIT5 (Pharmacia, Piscataway, NJ); pET-11d (Novagen, Madison, WI) Jameel et al., (1990) *J*. *Virol*. 64:3963-3966; and pSEM (Knapp et al. (1990) *BioTechniques* 8: 280-281). The use of pTRC, and pET-11d, for example, will lead to the expression of unfused protein. The use of pMAL, pRIT5 pSEM and pGEX will lead to the expression of allergen fused to maltose E binding protein (pMAL), protein A (pRIT5), truncated β-galactosidase (PSEM), or glutathione S-transferase (pGEX). When a *Lol p* V peptide of the invention is expressed as a fusion protein, it is particularly advantageous to introduce an enzymatic cleavage site at the fusion junction between the carrier protein and *Lol p* V peptide. The *Lol p* V peptide may then be recovered from the fusion protein through enzymatic cleavage at the enzymatic site and biochemical purification using conventional techniques for purification of proteins and peptides. Suitable enzymatic cleavage sites include those for blood clotting Factor Xa or thrombin for which the appropriate enzymes and protocols for cleavage are commercially available from, for example, Sigma Chemical Company, St. Louis, MO and N.E. Biolabs. Beverly, MA. The different vectors also have different promoter regions allowing constitutive or inducible expression with, for example. IPTG induction (PRTC, Amann et al., (1988) *supra*: pET-11d, Novagen, Madison, WI) or temperature induction (pRIT5, Pharmacia, Piscataway, NJ). It may also be appropriate to express recombinant *Lol p* V peptides in different *E*. *coli* hosts that have an altered capacity to degrade recombinantly expressed proteins (e.g. U.S. patent 4,758,512). Alternatively, it may be advantageous to alter the nucleic acid sequence to use codons preferentially utilized by E. coli, where such nucleic acid alteration would not affect the amino acid sequence of the expressed protein.

Host cells can be transformed to express the nucleic acid sequences of the invention using conventional techniques such as calcium phosphate or calcium chloride co-precipitation, DEAE-dextran-mediated transfection, or electroporation. Suitable methods for transforming the host cells may be found in Sambrook et al. *supra*, and other laboratory textbooks. The nucleic acid sequences of the invention may also be chemically synthesized using standard techniques (*i.e.* solid phase synthesis). Details of the isolation and cloning of clone 12R encoding *Lol p* V (described as *Lol p* Ib.1) are given in PCT application Publication Number WO 93/04174.

Nucleic acid sequences used in any embodiment of this invention can be cDNAs encoding the corresponding peptide sequences as shown in Fig. 7, or alternatively, can be any oligodeoxynucleotide sequence having all or a portion of a sequence represented herein, or their functional equivalents. Such oligodeoxynucleotide sequences can be produced chemically or mechanically, using known techniques. A functional equivalent of an oligonucleotide sequence is one which is 1) a sequence capable of hybridizing to a complementary oligonucleotide to which the sequence (or corresponding sequence portions) of *Lol p* V as shown in Fig. 1 (SEQ ID NO:1) or fragments thereof hybridizes, or 2) (the corresponding sequence portions complementary to the nucleic acid sequences encoding the peptide sequence derived from *Lol p* V as shown in Table 1 and Fig. 2 (SEQ ID NO:1), and/or Fig. 3) a sequence which encodes a product (e.g., a polypeptide or peptide) having the same functional characteristics of the product encoded by the sequence (or corresponding sequence portion) of *Lol p* V as shown in Fig. 1 (SEQ ID NO:1). Whether a functional equivalent must meet one or both criteria will depend on its use (e.g., if it is to be used only as an oligoprobe, it need meet only the first or second criteria and if it is to be used to produce a *Lol p* V peptide of the invention, it need only meet the third criterion).

A method of producing isolated *Lol p* V peptides of the invention or a portion thereof comprises the steps of culturing a host cell transformed with a nucleic acid sequence encoding a *Lol p* V peptide of the invention in an appropriate medium to produce a mixture of cells and medium containing said *Lol p* V peptide; and purifying the mixture to produce substantially pure *Lol p* V peptide. Host cells transformed with an expression vector containing DNA coding for a *Lol p* V peptide of the invention or a portion thereof are cultured in a suitable medium for the host cell. *Lol p* V peptides of the invention can be purified from cell culture medium, host cells, or both using techniques known in the art for purifying peptides and proteins including ion-exchange chromatography, gel filtration chromatography, ultrafiltration, electrophoresis and immunopurification with antibodies specific for the *Lol p* V peptides or portions thereof of the invention.

Antibodies specifically reactive with a *Lol p* V peptide may be used to standardize allergen extracts or to isolate the naturally occurring *Lol p*V . Also, *Lol p* V peptides of the invention can be used as "purified" allergens to standardize allergen extracts. For example, an animal such as a mouse or rabbit can be immunized with an immunogenic form of an isolated *Lol p* V peptide of the invention capable of eliciting an antibody response. Techniques for conferring immunogenicity on a peptide include conjugation to carriers or other techniques well-known in the art. The *Lot p* V peptide can be administered in the presence of adjuvant. The progress of immunization can be monitored by detection of antibody titers in plasma or serum standard ELISA or other immunoassay can be used with the immunogen as antigen to assess the levels of antibodies.

Following immunization, anti-*Lol p* V peptide antisera can be obtained and, if desired, polyclonal anti-*Lol p* V peptide antibodies from the serum. To produce monoclonal antibodies. antibody producing cells (lymphocytes) can be harvested from an immunized animal and fused by standard somatic cell fusion procedures with immortalizing cells such as myeloma cells to yield hybridoma cells. Hybridoma cells can be screened immunochemically for production of antibodies reactive with the *Lol p* V peptides of the invention. These sera of monoclonal antibodies can be used to standardize allergen extracts.

Through use of the peptides of the present invention preparations of consistent, well-defined composition and biological activity can be made and administered for therapeutic purposes (e.g. to modify the allergic response of a ryegrass pollen sensitive individual to pollen of such grasses or pollen of an immunologically related grass). Administration of such peptides may, for example, modify B-cell response to *Lol p* V allergen. T-cell response to *Lol p* V allergen or both responses. Isolated peptides can also be used to study the mechanism of immunotherapy of ryegrass pollen allergy and to design modified derivatives or analogues useful in immunotherapy.

T cell clones which specifically recognize *Lol p* V peptides of the invention may be suitable for isolation and molecular cloning of the gene for the T cell receptor which is specifically reactive with a peptide of the present invention. The T cell clones may be produced as described in Example 2, or as described in *Cellular Molecular Immunology,* Abul K. Abbas et al., W.B. Saunders Co. (1191) pg. 139. Soluble T cell receptors inhibit antigen-dependent activation of the relevant T cell subpopulation within an individual sensitive to *Lol p* V. Antibodies specifically reactive with such a T cell receptor can also be produced. Such antibodies may also be useful to block T-cell -MHC interaction in an individual. Methods for producing soluble T cell receptors are described in *Immunology; A Synthesis*, 2nd Ed., Edward S. Golub et al., Sinaur Assoc, Sunderland Massachusetts, (1991) pp. 366-369.

It is also possible to modify the structure of a peptide of the invention for such purposes as increasing solubility, enhancing therapeutic or preventive efficacy, or stability (e.g. shelf life ex vivo, and resistance to proteolytic degradation in vivo). A modified peptide can be produced in which the amino acid sequence has been altered, such as by amino acid substitution, deletion, or addition, to modify immunogenicity and/or reduce allergenicity, or to which a component has been added for the same purpose.

For example, a peptide can be modified so that it maintains the ability to induce T cell anergy and bind MHC proteins without the ability to induce a strong proliferative response or possibly, any proliferative response when administered in immunogenic form. In this instance, critical binding residues for the T cell receptor can be determined using known techniques (e.g., substitution of each residue and determination of the presence or absence of T cell reactivity). Those residues shown to be essential to interact with the T cell receptor can be modified by replacing the essential amino acid with another, preferably similar amino acid residue (a conservative substitution) whose presence is shown to enhance, diminish but not eliminate, or not affect T cell reactivity. In addition, those amino acid residues which are not essential for T cell receptor interaction can be modified by being replaced by another amino acid whose incorporation may enhance, diminish or not affect T cell reactivity but does not eliminate binding to relevant MHC.

Additionally, peptides of the invention can be modified by replacing an amino acid shown to be essential to interact with the MHC protein complex with another, preferably similar amino acid residue (conservative substitution) whose presence is shown to enhance, diminish but not eliminate, or not affect T cell activity. In addition, amino acid residues which are not essential for interaction with the MHC protein complex but which still bind the MHC protein complex can be modified by being replaced by another amino acid whose incorporation may enhance, not affect, or diminish but not eliminate T cell reactivity. Preferred amino acid substitutions for non-essential amino acids include, but are not limited to substitutions with alanine, glutamic acid, or a methyl amino acid.

In order to enhance stability and/or reactivity, peptides of the invention can also be modified to incorporate one or more polymorphisms in the amino acid sequence of the protein allergen resulting from natural allelic variation. Additionally, D-amino acids, non-natural amino acids or non-amino acid analogues can be substituted or added to produce a modified peptide within the scope of this invention. Furthermore, peptides of the present invention can be modified using the polyethylene glycol (PEG) method of A. Sehon and co-workers (Wie et al. *supra*) to produce a protein or peptide conjugated with PEG. In addition, PEG can be added during chemical synthesis of a protein or peptide of the invention. Modifications of peptides or portions thereof can also include reduction/ alyklation (Tarr in: *Methods of Protein Microcharacterization*, J.E. Silver ed. Humana Press. Clifton, NJ, pp 155-194 (1986)); acylation (Tarr, *supra*): chemical coupling to an appropriate carrier (Mishell and Shiigi, eds, *Selected Methods in Cellular Immunology*, WH Freeman, San Francisco, CA (1980): U.S. Patent 4,939,239; or mild formalin treatment (Marsh *International Archives of Allergy and Applied Immunology,* 41:199-215 (1971)).

To facilitate purification and potentially increase solubility of peptides of the invention, it is possible to add reporter group(s) to the peptide backbone. For example, poly-histidine can be added to a peptide to purify the peptide on immobilized metal ion affinity chromatography (Hochuli. E. et al., *Bio*/*Technology*, 6:1321-1325 (1988)). In addition, specific endoprotease cleavage sites can be introduced, if desired, between a reporter group and amino acid sequences of a peptide to facilitate isolation of peptides free of irrelevant sequences. In order to successfully desensitize an individual to a protein antigen, it may be necessary to increase the solubility of a peptide by adding functional groups to the peptide or by not including hydrophobic T cell epitopes or regions containing hydrophobic epitopes in the peptides or hydrophobic regions of the protein or peptide. It may also be necessary to replace hydrophobic residues with less hydrophobic residues, such changes affecting binding to a MHC complex or T cell receptor as described above.

To potentially aid proper antigen processing of T cell epitopes within a peptide, canonical protease sensitive sites can be recombinantly or synthetically engineered between regions, each comprising at least one T cell epitope. For example, charged amino acid pairs, such as KK or RR, can be introduced between regions within a peptide during construction of the peptide. The resulting peptide can be rendered sensitive to cathepsin and/or other trypsin-like enzymes cleavage to generate portions of the peptide containing one or more T cell epitopes. In addition, such charged amino acid residues can result in an increase in solubility of a peptide.

Site-directed mutagenesis of DNA encoding a peptide of the invention can be used to modify the structure of the peptide by methods known in the art. Such methods may, among others, include PCR with degenerate oligonucleotides (Ho et al., *Gene*, 77:51-59 (1989)) or total synthesis of mutated genes (Hostomsky, Z. et al., *Biochem. Biophys, Res. Comm.,* 161:1056-1063 (1989)). To enhance bacterial expression, the aforementioned methods can be used in conjunction with other procedures to change the eucaryotic codons in DNA constructs encoding protein or peptides of the invention to ones preferentially used in *E*. *coli*, yeast, mammalian cells, or other eukaryotic cells.

Peptides of the present invention can also be used for detecting and diagnosing ryegrass pollinosis. For example, this could be done by combining blood or blood products obtained from an individual to be assessed for sensitivity to ryegrass pollen with isolated peptides of *Lol p* V, under conditions appropriate for binding of components in the blood (e.g., antibodies, T-cells, B-cells) with the peptide(s) and determining the extent to which such binding occurs.

Isolated peptides of the invention when administered in a therapeutic regimen to a *Lol p* V-sensitive individual, or an individual allergic to an allergen cross-reactive with *Lol p* V, are capable of modifying the allergic response of the individual to *Lol p* V ryegrass pollen allergen or such cross -reactive allergen of the individual, and preferably are capable of modifying the B-cell response, T-cell response or both the B-cell and the T-cell and the T-cell response of the individual to the allergen. As used herein, modification of the allergic response of an individual sensitive to a ryegrass pollen allergen or cross- reactive allergen can be defined as non-responsiveness or diminution in symptoms to the allergen, as determined by standard clinical procedures (See e.g. Varney et al, *British Medical Journal*, 302:265-269 (1990)) including diminution in ryegrass pollen induced asthmatic symptoms. As referred to herein, a diminution in symptoms includes any reduction in allergic response of an individual to the allergen after the individual has completed a treatment regimen with a peptide or protein of the invention. This dimunition may be subjective (i.e. the patient feels more comfortable in the presence of the allergen). Dimunition in symptoms can be determined clinically as well, using standard skin tests as is known in the art.

*Lol p* V peptides of the present invention which have T cell stimulating activity, and thus comprise at least one T cell epitope are particularly desirable for therapeutic purposes. *Lol p* V peptides of the present invention which bind IgE and comprise at least one B cell epitope are particularly desirable for therapeutic purposes. In referring to an epitope, the epitope will be the basic element or smallest unit of recognition by a receptor, particularly immunoglobulins, histocompatibility antigens and T cell receptors where the epitope comprises amino acids essential to receptor recognition. Amino acid sequences which mimic those of the epitopes and which are capable of down regulating or reducing allergic response to *Lol p* V can also be used. T cell epitopes are believed to be involved in initiation and perpetuation of the immune response to a protein allergen which is responsible for the clinical symptoms of allergy. These T cell epitopes are thought to trigger early events at the level of the T helper cell by binding to an appropriate HLA molecule on the surface of an antigen presenting cell and stimulating the relevant T cell subpopulation. These events lead to T cell proliferation, lymphokine secretion, local inflammatory reactions, recruitment of additional immune cells to the site, and activation of the B cell cascade leading to production of antibodies. One isotype of these antibodies, IgE, is fundamentally important to the development of allergic symptoms and its production is influenced early in the cascade of events, at the level of the T helper cell, by the nature of the lymphokines secreted.

Exposure of ryegrass pollen allergic patients to isolated *Lol p* V peptides of the present invention which comprise at least one T cell epitope and are derived from *Lol p* V protein allergen may tolerize or anergize appropriate T cell subpopulations such that they become unresponsive to the protein allergen and do not participate in stimulating an immune response upon such exposure. In addition, administration of a peptide of the invention or portion thereof which comprises at least one T cell epitope may modify the lymphokine secretion profile as compared with exposure to the naturally-occurring *Lol p* V protein allergen or portion thereof (e.g. result in a decrease of IL-4 and/or an increase in IL-2). Furthermore, exposure to such peptide of the invention may influence T cell subpopulations which normally participate in the response to the naturally occurring allergen such that these T cells are drawn away from the site(s) of normal exposure to the allergen (e.g., nasal mucosa, skin. and lung) towards the site(s) of therapeutic administration of the fragment or protein allergen. This redistribution of T cell subpopulations may ameliorate or reduce the ability of an individual's immune system to stimulate the usual immune response at the site of normal exposure to the allergen, resulting in a dimunution in allergic symptoms.

The isolated *Lol p* V peptides of the invention can be used in methods of diagnosing, treating and preventing allergic reactions to *Lol p* V allergen or a cross reactive protein allergen. Thus, the present invention provides diagnostic compositions for diagnosing allergy to Lol p V allergen or a cross-reactive protein allergen. Such compositions may comprise synthetically or recombinantly produced peptides derived from Lol p V and a pharmaceutically acceptable carrier or diluent. Such diagnostic compositions of the invention may be used in diagnostic tests for allergy such as radio-allergosorbent test (RAST), paper radioimmunosorbent test (PRIST), enzyme linked immunosorbent assay (ELISA), radioimmunoassay (RI A), immuno-radiometric assay (IRMA), luminesience immunoassay (LIA), histamine release assays and IgE immonoblots. The present invention also provides therapeutic compositions comprising isolated *Lol p* V peptides or portions thereof produced in a host cell transformed to express such *Lol p* V peptide or portion thereof and a pharmaceutically acceptable carrier or diluent. The therapeutic compositions of the invention may also comprise synthetically prepared *Lol p* V peptides and a pharmaceutically acceptable carrier or diluent. Administration of the therapeutic compositions of the present invention to an individual to be desensitized can be carried out using known techniques. *Lol p* V peptides or portions thereof may be administered to an individual in combination with, for example, an appropriate diluent, a carrier and/or an adjuvant. Pharmaceutically acceptable diluents include saline and aqueous buffer solutions. Pharmaceutically acceptable carriers include polyethylene glycol (Wie et al. (1981) *Int. Arch. Allergy Appl. Immunol.* 64:84-99) and liposomes (Strejan et al. (1984) *J. Neuroimmunol* 7: 27). For purposes of inducing T cell anergy, the therapeutic composition is preferably administered in nonimmunogenic form, e.g. it does not contain adjuvant. The therapeutic compositions of the invention are administered to ryegrass pollen-sensitive individuals or individuals sensitive to an allergen which is immunologically cross-reactive with ryegrass pollen allergen..

Administration of the therapeutic compositions of the present invention to an individual to be desensitized can be carried out using known procedures at dosages and for periods of time effective to reduce sensitivity (i.e., reduce the allergic response) of the individual to the allergen. Effective amounts of the therapeutic compositions will vary according to factors such as the degree of sensitivity of the individual to ryegrass pollen, the age, sex, and weight of the individual, and the ability of the protein or fragment thereof to elicit an antigenic response in the individual.

The active compound (i.e., protein or fragment thereof) may be administered in a convenient manner such as by injection (subcutaneous, intravenous, etc.), oral administration, inhalation, transdermal application, or rectal administration. Depending on the route of administration, the active compound may be coated within a material to protect the compound from the action of enzymes, acids and other natural conditions which may inactivate the compound.

For example, preferably about 1 µg- 3 mg and more preferably from about 20-500 µg of active compound (i.e., protein or fragment thereof) per dosage unit may be administered by injection. Dosage regimen may be adjusted to provide the optimum therapeutic response. For example, several divided doses may be administered daily, weekly, monthly, quarterly, or yearly as needed or the dose may be proportionally reduced or increased as indicated by the exigencies of the therapeutic situation.

To administer a peptide by other than parenteral administration, it may be necessary to coat the protein with, or co-administer the protein with, a material to prevent its inactivation. For example, peptide or portion thereof may be co-administered with enzyme inhibitors or in liposomes. Enzyme inhibitors include pancreatic trypsin inhibitor, diisopropylfluorophosphate (DEP) and trasylol. Liposomes include water-in-oil-in-water CGF emulsions as well as conventional liposomes (Strejan *et al*., (1984) J. Neuroimmunol. 7:27).

The active compound may also be administered parenterally or intraperitoneally. Dispersions can also be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions of dispersion. In all cases, the composition must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing. for example, water, ethanol, polyol (for example, glyceral, propylene glycol, and liquid polyetheylene glycol, and the like), suitable mixtures thereof, and vegetable oils. The proper fluidity can be maintained, for example, by the use of a coating such as lecithin by the maintenance of the required particle size in the case of dispersion and by the use of surfactants. Prevention of the action of microorganisms can be achieved by various antibacterial and antifungal agents, for example, parabens. chlorobutanol, phenol, ascorbic acid, thiomersal, and the like. In many cases, it will be preferable to include isotonic agents, for example, sugars, polyalcohols such as mannitol and sorbitol or sodium chloride in the composition. Prolonged absorption of the injectable compositions can be brought about, including in the composition, an agent which delays absorption, for example, aluminum monostearate and gelatin.

Sterile injectable solutions can be prepared by incorporating active compound (i.e.. protein or peptide) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (i.e., protein or peptide) plus any additional desired ingredient from a previously sterile-filtered solution thereof.

When a peptide of the invention is suitably protected, as described above, the peptide may be orally administered. for example, with an inert diluent or an assimilable edible carrier. The peptide and other ingredients may also be enclosed in a hard or soft shell gelatin capsule, compressed into tablets, or incorporated directly into the individual's diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the composition and preparations may, of course, be varied and may conveniently be between about 5 to 80% of the weight of the unit. The amount of active compound in such therapeutically useful compositions is such that a suitable dosage will be obtained. Preferred compositions or preparations according to the present invention are prepared so that an oral dosage unit contains between from about 10 µg to about 200 mg of active compound.

The tablets, troches, pills, capsules and the like may also contain the following: a binder such as gum gragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservative, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier" includes any and all solvents. dispersion media, coatings, antibacterial and antifungal agents, isotonic and absorption delaying agents. and the like. The use of such media and agents for pharmaceutically active substances is well known in the art. Except insofar as any conventional media or agent is incompatible with the active compound, use thereof in the therapeutic compositions is contemplated. Supplementary active compounds can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated: each unit containing a predetermined quantity of active compound calculated to produce the desired therapeutic effect in association with the required pharmaceutical carrier. The specification for the novel dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Various isolated peptides of the invention derived from ryegrass pollen protein *Lol p* V are shown in Fig. 2 and in Table 1. Peptides comprising at least two regions, each region comprising at least one T cell epitope of *Lol p* V are also within the scope of the invention. As used herein a region may include the amino acid sequence of a peptide of the invention as shown in Fig. 2 or the amino acid sequence of a portion of such peptide.

To obtain isolated peptides of the present invention. *Lol p* V is divided into non-overlapping peptides of desired length or overlapping peptides of desired lengths as discussed in Example 1 which can be produced recombinantly, or synthetically. Peptides comprising at least one B cell epitope are capable of binding IgE. Peptides comprising at least one T cell epitope are capable of eliciting a T cell response, such as T cell proliferation or lymphokine secretion, and/or are capable of inducing T cell anergy (i.e., tolerization). To determine peptides comprising at least one T cell epitope, isolated peptides are tested by, for example. T cell biology techniques, to determine whether the peptides elicit a T cell response or induce T cell anergy. Those peptides found to elicit a T cell response or induce T cell anergy are defined as having T cell stimulating activity.

As discussed in Example 2, human T cell stimulating activity can be tested by culturing T cells obtained from an individual sensitive to *Lol p* V allergen, (i.e., an individual who has an IgE mediated immune response to *Lol p* V allergen) with a peptide derived from the allergen and determining whether proliferation of T cells occurs in response to the peptide as measured, e.g., by cellular uptake of tritiated thymidine. Stimulation indices for responses by T cells to peptides can be calculated as the maximum CPM in response to a peptide divided by the control CPM. A stimulation index (S.I.) equal to or greater than two times the background level is considered "positive". Positive results are used to calculate the stimulation index for each peptide for the patient tested. Preferred peptides of this invention comprise at least one T cell epitope and have a T cell stimulation index of greater than or equal to 2.0. A peptide having a T cell stimulation index of greater than or equal to 2.0 in a ryegrass pollen sensitive patient is considered useful as a therapeutic agent. Preferred peptides have a T cell stimulation index of at least 2.2, more preferably at least 2.5, more preferably at least 3.0, more preferably at least 3.4. For example, peptides of the invention having a T cell stimulation index of at least 2.5, as shown in Figs 4, 5, or 6, include peptides 19 (SEQ ID NO:21), 22 (SEQ ID NO:24), and 34 (SEQ ID NO:36).

In order to determine precise T cell epitopes by, for example, fine mapping techniques, a peptide having T cell stimulating activity and thus comprising at least one T cell epitope as determined by T cell biology techniques is modified by addition or deletion of amino acid residues at either the amino or carboxy terminus of the peptide and tested to determine a change in T cell reactivity to the modified peptide. Following this technique, peptides are selected and produced recombinantly or synthetically. Preferred therapeutic peptides are selected based on various factors, including the strength of the T cell response to the peptide (e.g., stimulation index), the frequency of the T cell response to the peptide in a population of individuals sensitive to ryegrass pollen, and the potential cross-reactivity of the peptide with other allergens from other species of grasses as discussed earlier. The physical and chemical properties of these selected peptides (e.g., solubility, stability) are examined to determine whether the peptides are suitable for use in therapeutic compositions or whether the peptides require modification as described herein. The ability of the selected peptides or selected modified peptides to stimulate human T cells (e.g., induce proliferation, lymphokine secretion) is determined.

Additionally, preferred T cell epitope-containing therapeutic peptides of the invention do not bind immunoglobulin E (IgE) or bind IgE to a substantially lesser extent than the protein allergen from which the peptide is derived. The major complications of standard immunotherapy are IgE-mediated responses such as anaphylaxis. Immunoglobulin E is a mediator of anaphylactic reactions which result from the binding and cross-linking of antigen to IgE on mast cells or basophils and the release of mediators (e.g., histamine, serotonin, eosinophil chemotacic factors). Thus, anaphylaxis in a substantial percentage of a population of individuals sensitive to *Lol p* V could be avoided by the use in immunotherapy of a peptide or peptides which do not bind IgE in a substantial percentage (e.g., at least about 75%) of a population of individuals sensitive to *Lol p* V allergen, or if the peptide binds IgE, such binding does not result in the release of mediators from mast cells or basophils. The risk of anaphylaxis could be reduced by the use in immunotherapy of a peptide or peptides which have reduced IgE binding. Moreover, peptides which have minimal IgE stimulating activity are desirable for therapeutic effectiveness. Minimal IgE stimulating activity refers to IgE production that is less than the amount of IgE production and/or IL-4 production stimulated by the native *Lol p* V protein allergen.

A T cell epitope containing peptide of the invention, when administered to a ryegrass pollen-sensitive individual in a therapeutic treatment regimen is capable of modifying the allergic response of the individual to the allergen. Particularly, *Lol p* V peptides of the invention comprising at least one T cell epitope of *Lol p* V or at least two regions derived from *Lol p* V, each comprising at least one T cell epitope, when administered to an individual sensitive to ryegrass pollen are capable of modifying T cell response of the individual to the allergen.

An isolated *Lol p* V therapeutic peptide of the invention comprises at least one T cell epitope of the *Lol p* V and accordingly the peptide comprises at least approximately seven amino acids residues. For purposes of therapeutic effectiveness, preferred therapeutic compositions of the invention preferably comprise at least two T cell epitopes of *Lol p* V, and accordingly, the peptide comprises at least approximately eight amino acid residues and preferably at least fifteen amino acid residues. Additionally, therapeutic compositions comprising preferred isolated peptides of the invention preferably comprise a sufficient percentage of the T cell epitopes of the entire protein allergen such that a therapeutic regimen of administration of the composition to an individual sensitive to ryegrass pollen, results in T cells of the individual being tolerized to the protein allergen. Synthetically produced peptides of the invention comprising up to approximately forty-five amino acid residues in length, and most preferably up to approximately thirty amino acid residues in length are particularly desirable as increases in length may result in difficulty in peptide synthesis. Peptides of the invention may also be produced recombinantly as described above, and it is preferable that peptides of 45 amino acids or longer be produced recombinantly.

Peptides derived from the *Lol p* V protein allergen which can be used for therapeutic purposes comprise at least one T cell epitope of Lol p V and comprise all or a portion of the amino acid sequences of the following peptides: 6 (SEQ ID NO:8), 7 (SEQ ID NO:9), 12 (SEQ ID NO:14), 16 (SEQ ID NO:18), 19 (SEQ ID NO:21), 22 (SEQ ID NO:24), 28 (SEQ ID NO:30), 33 (SEQ ID NO:35), and 34 (SEQ ID NO:36) (as shown in fig.2) preferably, the peptide comprising an amino acid sequence of peptides: 6 (SEQ ID NO:8), 7 (SEQ ID NO:9), 12 (SEQ ID NO:14), 16 (SEQ ID NO:18), 19 (SEQ ID NO:21), 22 (SEQ ID NO:24), 28 (SEQ ID NO:30), 33 (SEQ ID NO:35), and 34 (SEQ ID NO:36) (as shown in Fig. 2) has a mean T cell stimulation index equivalent to, or greater than the mean T cell stimulation index of the peptide from which it is derived as shown in Figs. 4, 5 or 6. Even more preferably peptides derived from the *Lol p* V protein allergen which can be used for therapeutic purposes comprise all or a portion of the amino acid sequences of the following peptides: 19 (SEQ ID NO:21), 22 (SEQ ID NO:24), and 34 (SEQ ID NO:36) as shown in Fig. 2.

In one aspect of the present invention, a composition is provided comprising at least two peptides (e.g., a physical mixture of at least two peptides), each comprising at least one T cell epitope of *Lol p* V. Such compositions can be administered in the form of a therapeutic composition with a pharmaceutically acceptable carrier or diluent. Additionally, therapeutically effective amounts of one or more of therapeutic compositions and comprising at least one peptide having a T cell epitope can be administered simultaneously or sequentially to an individual sensitive to ryegrass pollen.

Preferred compositions and preferred combinations *of Lol p* V peptides which can be administered simultaneously or sequentially (comprising peptides which comprise amino acid sequences shown in Fig. 2) include the following combinations:
14 (SEQ ID NO:16) and 22 (SEQ ID NO:24);
19 (SEQ ID NO:21) and 22 (SEQ ID NO:24);
19 (SEQ ID NO:21) and 25 (SEQ ID NO:27);
14 (SEQ ID NO:16), 22 (SEQ ID NO:24), and 25 (SEQ ID NO:27);
19 (SEQ ID NO:21), 22 (SEQ ID NO:24), and 25 (SEQ ID NO:27);
14 (SEQ ID NO:16), 22 (SEQ ID NO:24), 25 (SEQ ID NO:27), and 34 (SEQ ID NO:36);
19 (SEQ ID NO:21), 22 (SEQ ID NO:24), 25 (SEQ ID NO:27), and 34 (SEQ ID NO:36);
14 (SEQ ID NO:16), 19 (SEQ ID NO:21), 22 (SEQ ID NO:24), and 25 (SEQ ID NO:27);
14 (SEQ ID NO:16), 19 (SEQ ID NO:21), 22 (SEQ ID NO:24), 25 (SEQ ID NO:27), and 34 (SEQ ID NO:36);
6 (SEQ ID NO:8), 7 (SEQ ID NO:9), 9 (SEQ ID NO:11), 12 (SEQ ID NO:14), 14 (SEQ ID NO:16), 16 (SEQ ID NO:18), 19 (SEQ ID NO:21), 22 (SEQ ID NO:24), 25 (SEQ ID NO:27), 33 (SEQ ID NO:27), 33 (SEQ ID NO:35), and 34 (SEQ ID NO:36);
6 (SEQ ID NO:8), 9 (SEQ ID NO:11), 16 (SEQ ID NO:18), 19 (SEQ ID NO:21), 22 (SEQ ID NO:24), and 34 (SEQ ID NO:36); and
6 (SEQ ID NO:8), 9 (SEQ ID NO:11), 14 (SEQ ID NO:16), 16 (SEQ ID NO:18), 19 (SEQ ID NO:21), 22 (SEQ ID NO:24), 25 (SEQ ID NO:27), and
34 (SEQ ID NO:36).

### EXAMPLES

### EXAMPLE 1

### Lol p V peptides

The amino acid sequence of *Lol p* V (SEQ ID NO:2) was deduced from the cDNA sequence of clone 12R (SEQ ID NO:1) ATCC Number as shown in Fig. 1. The cloning and sequencing of clone 12R encoding *Lol p* V (described as *Lol p* Ib.1) is described in PCT application publication number WO 93/04174. Thirty 12-mer and four 13-mer solid phase peptides were synthesized on a BT7400 manual peptide synthesising block (Biotech Instruments Ltd, UK). These peptides corresponded to the entire *Lol p* V sequence with four (or five) amino acid overlap. The peptides were synthesized on C-teminal Fmoc protected amino acid resins (25 mM Auspep, Australia). The resins were washed and drained sequentially with 3 x 1 ml dimethylformamide (DMF: Auspep, Australia), and then treated with 1ml deprotecting solution (DP, 25% w/v piperidine in DMF; Auspep, Australia) for ten minutes to remove the Fmoc group from the first C-terminal amino acid. The wells were then washed and drained sequentially with 3 x 1 ml DMF, 2 x 1ml dichloromethane (DCM; Auspep, Australia) and finally 2 x 1 ml DMF. Ten fold excess of the selected amino acids were combined with activating reagent (BOP:HOBYT 1:1; Auspep, Australia), dissolved in 1 ml activating solution (Auspep, Australia) and added to the appropriate wells for coupling. The procedure was repeated until the desired peptide length was achieved.

The peptides were then treated with cleaving solution (90% v/v trifluoroacetic acid, 4% v/v phenol, 1% v/v ethanedithiol; Auspep, Australia) for six hours to facilitate peptide cleavage from the wells. The collected peptides were then precipitated in 50 ml cold diethylether (DEE: Auspep, Australia) and then isolated on Whatman's filter paper 1 (Whatman, UK). These filter papers were washed with 50 ml lyophilising solution (Auspep, Australia) and freeze dried. Peptides were subjected to analysis by HPLC and mass spectromety by Auspep Laboratories to check sequence and peptide purity.

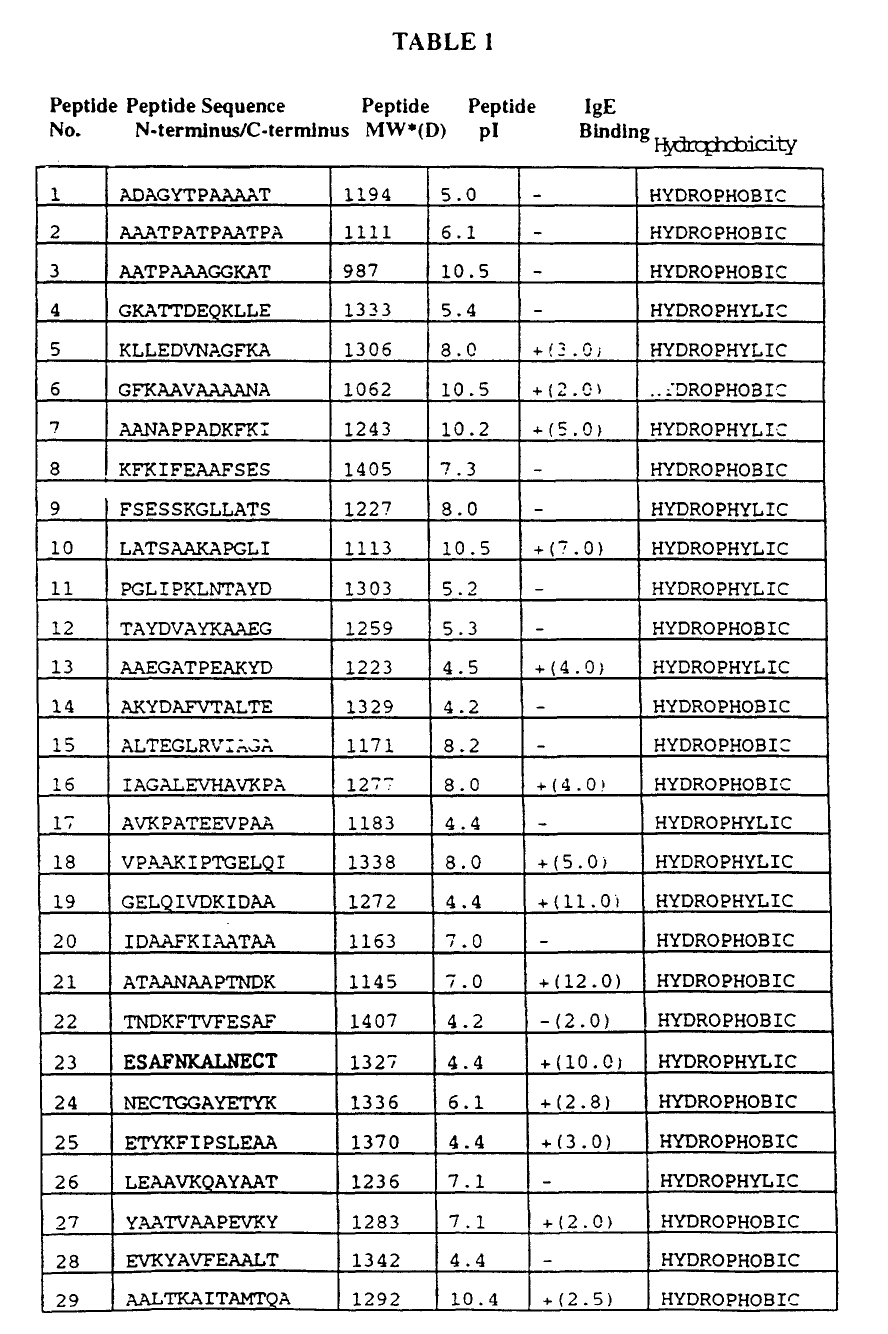

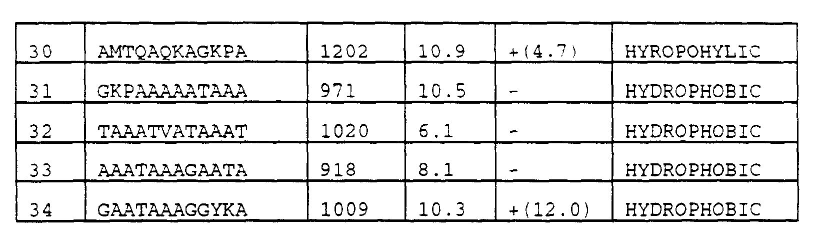

### Peptide Dot Blot Immunoassays

The peptides were dissolved in water to give a 1.6 mM stock solution. Insoluble peptides were dissolved by sonication (Branson Sofifier 450. USA) for one minute.

The peptides were immobilized onto nitrocellulose (NC) filter by using a modified method published elsewhere (Sithigonglu (1991) J. Immunol. Methods, 141:23-32). Two µl of the peptide stock solutions were spotted on the NC filter (Schleicher & Schuell, W. Germany) about 1 cm apart. After the strips were dried and baked at 80°C for one hour, they were fixed by exposure, in a tightly sealed plastic box, to vapor from 0.2% glutaraldehyde in PBS at room temperature (18°C) for one hour. After washing thoroughly with distilled water, the NC filters were blocked with Blotto (10% non-fat dry milk in PBS) for two hours and washed in 1X Tween-PBS (0.1% Tween-20 in PBS) and 2X PBS, five minutes each.

The peptides were then screened for human IgE and mouse/rabbit IgG epitopes with individual sera (diluted 1:4 in PBS with 0.5% BSA). MAbs FMC A7 (Smart et al. ( 1983) Int. Arch. Allergy Clin. Immunol. 72:243) and polyclonal rabbit anti-Lol p V (Lol p Ib) antibody.

Out of the fifty highly ryegrass pollen allergic subjects, twenty-three had IgE that bound to at least one peptide. Although the majority of the subjects showed mild to moderate IgE-reactivity, there were several showing high reactivity. The dot blot analysis for IgE binding is shown in Fig. 7.

The intensity of each dot blot was measured by densitometer (Pharmacia LKB Ultra Scan XL, Sweden). Values are given in arbitrary densitometric units and shown in Fig. 8. Densitometric values greater than two were considered as positive binding in comparison to the background.

For every peptide, the sera and antibody values have been added (score/peptide) and divided by the number of positive sera to express the final value as an average (score/sera). Sera C1 and C2 were not grass allergic and were used as negative controls. C refers to crude ryegrass pollen extract (1µg/dot) as positive control (see example 3).

Sera 1, 14, 36, 42 and 48 indicated substantial affinity towards a selection of specific peptides (e.g. peptides 19 (SEQ ID NO:21), 34 (SEQ ID NO:36), and 7 (SEQ ID NO:9)), which were classified as immunodominant linear allergenic B-cell epitopes of Lol p V (Figures 7 and 8).

Peptide numbers 7 (SEQ ID NO:9), 16 (SEQ ID NO:18), 19 (SEQ ID NO:21), 21 (SEQ ID NO:23), 23 (SEQ ID NO:25), 30 (SEQ ID NO:32) and 34 (SEQ ID NO:36) indicated the highest human IgE-reactivity and were thus classified as major linear allergenic B-cell epitopes of Lol p V. MAb FMC-A7, and rabbit polyclonal anti-Lol p Ib antiserum were analyzed for IgG binding to the peptides. This data is shown in Fig. 7. The MAbs and rabbit antiserum generally showed low binding to the peptides. Peptide numbers 1 (SEQ ID NO:3), 7 (SEQ ID NO:9), 19 (SEQ ID NO:21), 23 (SEQ ID NO:25), 24 (SEQ ID NO:26) and 28 (SEQ ID NO:30) indicated the highest mouse/rabbit IgG-reactivity.

### EXAMPLE 2

### 1. MATERIALS AND METHODS

### Crude Rye and Bermuda Grass Pollen Extracts

Rye grass (Lolium perenne) and Bermuda grass (Cynodon dactylon) pollen was obtained from Greer Laboratories (USA) as dry non-defatted extracts. Pollen allergens were extracted in 0.001M NH₄HCO₃ (10% w/v), centrifuged and the supernatants dialysed against Phosphate buffered saline (PBS) overnight. The supernatants were then passed through a 0.20 µm filter and protein concentration was determined using the BioRad Microassay (Bio-Rad, USA) according to the manufacturer's instructions.

### Lol p V

Lol p V was purified from crude rye grass pollen extracts by fluid phase liquid chromatography (FPLC). Briefly, the crude rye grass pollen extract was subjected to a buffer change from PBS to 20 mM Tris pH12 (buffer A) on an Econo-Pac 10DG column (Bio-Rad, USA). This extract, 5 mg, was injected onto an ion-exchange FPLC column (Mono Q HR 5/5; Pharmacia, Sweden), pre-equilibrated sequentially with buffer A for five minutes, then buffer B (20mM Tris, pH 7.0) for ten minutes and finally with buffer A for a further five minutes. The column was washed with buffer A for ten minutes to allow for the binding of Lol p V as well as for the elution of other proteins with pI values equal to or higher than twelve. Elution of Lol p V was performed with buffer B at a constant flow rate of 1 ml/minute for fifteen minutes at room temperature. Fractions of 1ml were collected every minute and then analyzed by SDS-PAGE and immunoblotting. Fractions containing Lol p V were pooled and the protein concentration was determined.

### Lol p V peptides

The same Lol p V peptides were produced as described in Example 1. Peptide 13 (SEQ ID NO:15) was produced in very small amounts only and could not be tested in this study.

### Generation of allergen-reactive T cell lines and clones

Peripheral blood mononuclear cells (PBMC) from a healthy adult with a positive skin test to rye grass pollen, were isolated from heparinised blood samples by centrifugation on a Ficoll-Paque (Pharmacia, Sweden) density gradient. Initially 1 x 10⁶/ml PBMC were stimulated with 5 µg/ml of Lol p V in a 96-well U-bottomed microtiter plates (Linbro, USA). All cells were cultured in RPM1-1640 (ICN, Australia) tissue culture medium supplemented with 1% w/v L-glutamine (ICN, Australia), 0.125% w/v gentomicin (Delta West, Australia) and 5% v/v heat-inactivated human A+ serum. Cultures were performed at 37°C in 5% v/v CO₂ atmosphere.

Seven days after initiation of culture, ten units/ml of human recombinant IL-2 (rIL-2; Cetus, USA) was added to the wells. On day 14 the T cell line was expanded by transferring the cells to a 24-well tissue culture plate (Costar, USA) and adding 5 x 10⁵/ml irradiated (2000 rads) autologous PBMC, 2.5 µg/ml Lol p V, 10-20 units/ml rIL-2, 5-10 units/ml Lymphocult (Biotest, Germany) and 0.1µg/ml PHA (Wellcome, UK). The line was restimulated every 8-10 days as described.

T cells were then cloned by limiting dilution of the line. For this, T cells were seeded at 300, 30, 3, 1 and 0.3 cells/well in 96-well U-bottomed microtiter plates together with 1x10⁶/ml irradiated PBMC, 2.5 µg/ml Lol p V, 20 units/ml rIL-2, 10 units/ml Lymphocult and 0.1 µg/ml PHA. The clones were maintained with rIL-2 every four days and irradiated autologous PBMC, Lol p V, rIL-2 and Lymphocult were added every 8-10 days.

Before use in proliferation assays the lines and clones were rested for 7-8 days after the last addition of feeder cells, allergen and IL-2.

### Proliferation assays

5x10⁵/ml T cells were cultured with 5x10⁵/ml irradiated autologous PBMC and Lol p V (0.019-2.5 µg/ml or 0.625-20µg/ml) or Lol p V peptides (1 and 10µg/ml) in 96-well U-bottomed microtiter plates for 88 hours, then pulsed with 1µCi/well tritiated methyl thymidine ([H]TdR; Amersham, USA) for eight hours prior to harvesting onto glass-fibre filters. Control cultures included cells with no allergen, cells with PHA, T cells alone and irradiated PBMC cells alone. The counts per minute (CPM) for each filter was determined using a Liquid Scintillation counter (LKB Wallace, Finland).

### Immunophenotyping

T cells were stained by direct or indirect immunofluorescence using the following monoclonal antibodies: CD2, CD3.CD4, CD5, CD8, CD11b, CD16, CD19, CD25, CD45RO, CD45RA, MHC class II, TcRαβ, TcRγδ, k light chain and λ light chain. For indirect immunofluorescence, FITC-conjugated sheep-anti-mouse Ig F(ab)₂ (Silenus, Australia) was used. Cells were then analyzed using a FACScan flow cytometer (Becton-Dickinson, USA).

### 2. RESULTS

### Allergen reactivity of the T cell line

On day 34 of culture, the T cell line was tested for reactivity to rye and Bermuda grass pollen allergens. Over a range of concentrations there was a strong proliferative response to the inducing allergen Lol p V and there was a lower response to crude rye grass pollen extract (Fig. 3). There was no response to the crude Bermuda grass pollen extract.

### Reactivity of the T cell line and clones to Lol p V synthetic peptides

The T cell line was first tested for reactivity to the Lol p V synthetic peptides on day 14, but no significant response was detected. However, at day 27, a strong response to peptide 14 (SEQ ID NO:16) was observed. By day 49, the strong reactivity to peptide 14 (SEQ ID NO:16) was maintained but there was additional weaker response to peptide 25 (SEQ ID NO:27) (Fig. 4), consistent with the polyclonal nature of the T cell line. The amino acid sequence of peptides 14 (SEQ ID NO:16) and 25 (SEQ ID NO:27) are shown in Table 2.

T cell clones were generated from the T cell line at day 17. One of the eight T cell clones obtained, designated A12, showed a strong response to peptide 14 (SEQ ID NO:16) only (Fig. 5). The stimulation index of the clone A12 response to peptide 14 (SEQ ID NO:16) was 137, compared with 4.8 for the T cell line at day 49, correlating with selection of peptide 14-specific T cells.

### Phenotypic analysis of the T cell line

The phenotype of the T cell line at day 47 was analyzed by flow cytometry using a panel of monoclonal antibodies against cell surface markers. The line expressed CD2, CD3, CD5, CD4, CD25, CD45RO, MHC class II and TcRαβ, consistent with the phenotype of activated mature helper T cells.

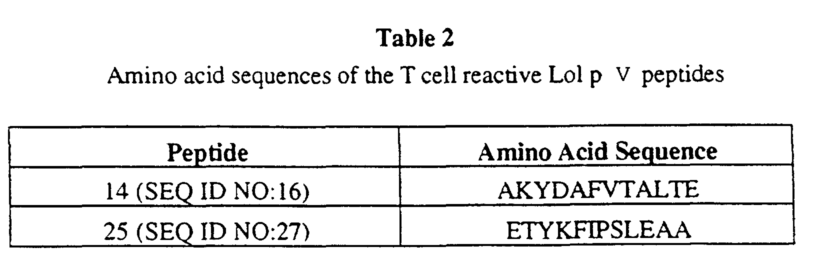

### EXAMPLE 3

### 1. MATERIALS AND METHODS

Another T cell line was produced from the same patient described in Example 2 in accordance with the procedure discussed in Example 2. Proliferation assays were conducted using the *Lol p* V synthetic peptides described in Example 1 in accordance with the procedure discussed in Example 2.

### 2. RESULTS

The T cell line was tested for reactivity to the *Lol p* V synthetic peptides The results are shown in Fig. 6. Proliferation is given with background proliferation subtracted. Background proliferation (proliferation to medium alone) was 2500 cpm. Stimulation indexes were calculated from the results shown by adding 2500 cpm to the values shown in Fig. 6 and dividing by 2500. Peptides 6 (SEQ ID NO:8), 7 (SEQ ID NO:9), 9 (SEQ ID NO:11), 12 (SEQ ID NO:14), 16 (SEQ ID NO:18), 19 (SEQ ID NO:21), 22 (SEQ ID NO:24), 28 (SEQ ID NO:30), 33 (SEQ ID NO:35), and 34 (SEQ ID NO:36) had stimulation indexes of greater than or equal to 2.0 as shown in Fig. 6. The most reactive peptides are 19 (SEQ ID NO:21), 22 (SEQ ID NO:24), and 34 (SEQ ID NO:36), with respective T cell stimulation indexes of 2.9, 3.4 and 2.5.

### EXAMPLE 4

IgE BINDING STUDIES WITH PEPTIDE 34 USING INHIBITION PEPTIDE DOT BLOTS AND INHIBITION ELISA

### A. Inhibition peptide dot-blots.

### Brief methodology:

Peptides 34 (SEQ ID NO:36), 6 (SEQ ID NO:8), 24 (SEQ ID NO:26) and 31 (SEQ ID NO:33) (last three peptides used as negative controls) as well as FPLC purified *Lol p* V and crude ryegrass pollen extract (designated as IX and C, respectively, in Fig. 9 and used as positive controls) were immobilized on NC, baked and gluteraldehyde fixed as previously described. Two separate lots of serum (from subject Girgis possessing peptide 34-specific IgE antibodies; diluted 1:4) were preincubated overnight at RT (18° C) either with 50 µg crude ryegrass pollen extract per ml diluted serum (designated as inhibited serum on the figure) or without the crude extract (designated as control serum). Peptide blots were then separately incubated in the control and the crude extract preincubated serum overnight followed by ¹²⁵I labelled anti-human IgE antibodies and exposed to X-ray film for up to 96 hrs.

### Results:

The control serum gave strong IgE-binding to peptide 34 (SEQ ID NO:36) and the positive controls *Lol p* IX and crude ryegrass pollen extract. The other control peptides (e.g. alanine-rich peptide 31 (SEQ ID NO:33), and peptides 6 (SEQ ID NO:8), 24 (SEQ ID NO:26)) gave no IgE-binding.

The serum preincubated with crude ryegrass pollen extract gave a reduced IgE-binding to peptide 34 (SEQ ID NO:36) as well as to the positive controls *Lol p* V and the crude ryegrass pollen extract.

### Conclusions:

These results demonstrate that this individual has peptide 34-specific IgE antibodies which can be inhibited by crude ryegrass pollen extract.

### B. Inhibition ELISA.

### Brief methodology:

Peptides 34 (SEQ ID NO:36), 6 (SEQ ID NO:8), 24 (SEQ ID NO:26) and 31 (SEQ ID NO:33) (last three peptides used as negative controls) and crude ryegrass pollen extract (used as positive control) were coated on microtiter plates. Serum of the individual (diluted 1:4) was preincubated either with or without different concentrations of crude ryegrass pollen extract overnight at RT. The peptide/crude extract coated wells were then incubated with the control and crude extract preincubated serum at the different crude extract concentrations. Plates were washed and incubated with anti-human IgE raised in rabbit followed by HRP-conjugated anti-rabbit antibodies. The plates were devleoped with the peroxidase substrate 1, 2-Phenylenediamine and the colour reaction was read at 492nm.

### Results:

As shown in Fig. 10, the control serum gave IgE-binding to peptide 34 (SEQ ID NO:36) and crude ryegrass pollen extract. The gradual increase in the concentration of crude extract gave a gradual decrease in IgE-binding to both peptide 34 (SEQ ID NO:36) and crude extract. The control peptides all displayed a significantly lower IgE-binding and there was no significant correlation between the gradual decrease in IgE-binding with increasing inhibitor concentration. The binding shown by the control peptides is due to background, that is non-specific IgE-binding to the peptides or the well due to assay conditions. However, even though a slight background was observed with ELISA, the IgE-binding to peptide 34 (SEQ ID NO:36) and crude extract was higher than the control peptides.

### Conclusions:

As with the inhibition dot-blot results, the inhibition ELISA results indicates significant IgE-binding to peptide 34 (SEQ ID NO:36) in this individual.

## Claims

1. An isolated peptide fragment of Lol pV or a homologue thereof consisting of an amino acid sequence selected from amino acid sequences as shown in Fig 2 of peptides 6 (SEQ ID NO:8), 7 (SEQ ID NO:9), 9 (SEQ ID NO:11), 12 (SEQ ID NO:14), 16 (SEQ ID NO:18), 19 (SEQ ID NO:21), 22 (SEQ ID NO:24), 28 (SEQ ID NO:30), 33 (SEQ ID NO:35) and 34 (SEQ ID NO:36).

2. An isolated peptide fragment as claimed in claim 1, wherein said peptide fragment comprises at least one B cell epitope of Lol p V, selected from the group consisting of amino acid sequences as shown in Fig. 2 of peptides 7 (SEQ ID NO:9), 16 (SEQ ID NO:18), 19 (SEQ ID NO:21), 28 (SEQ ID NO:30) and 34 (SEQ ID NO:36).

3. An isolated peptide fragment as claimed in claim 1 or 2 which, when administered to an individual sensitive to *Lol p* V allergen, induces T cell anergy in the individual or modifies the lymphokine secretion profile of T cells in the individual.

4. An isolated peptide fragment as claimed in claim 2 or claim 3 which either:-
(a) does not bind immunoglobulin E specific for *Lol p* V in at least about 75% of individuals sensitive to *Lol p* V, or if binding of the peptide or portion thereof to said immunoglobulin E occurs, such binding does not result in release of mediators from mast cells or basophils in a substantial percentage of individuals sensitive to *Lol p* V; or
(b) binds immunoglobulin E to a lesser extent than purified native *Lol p* V binds immunoglobulin E.

5. A peptide fragment as claimed in any one of claims 1 to 4 wherein said peptide fragment is produced by chemical synthesis

6. An isolated nucleic acid consisting of a sequence encoding a peptide of any one of claims 1 to 5.

7. An isolated peptide fragment as claimed in claims 1 to 4, produced in a host cell transformed with a nucleic acid of claim 6.

8. An expression vector encoding a peptide fragment of claims 1 to 4.

9. A therapeutic composition comprising at least one isolated peptide of any one of claims 1 to 5 or 7 and a pharmaceutically acceptable carrier or diluent.

10. A composition of claim 9 comprising a combination of peptides selected from:
14 (SEQ ID NO:16) and 22 (SEQ ID NO:24);
19 (SEQ ID NO:21) and 22 (SEQ ID NO:24);
19 (SEQ ID NO:21) and 25 (SEQ ID NO:27);
14 (SEQ ID NO:16), 22 (SEQ ID NO:24), and 25 (SEQ ID NO:27);
19 (SEQ ID NO:21), 22 (SEQ ID NO:24), and 25 (SEQ ID NO:27);
14 (SEQ ID NO:16), 22 (SEQ ID NO:24), 25 (SEQ ID NO:27, and 34 (SEQ ID NO:36);
19 (SEQ ID NO:21), 22 (SEQ ID NO:24), 25 (SEQ ID NO:27), and 34 (SEQ ID NO:36);
14 (SEQ ID NO:16), 19 (SEQ ID NO:21), 22 (SEQ ID NO:24), and 25 (SEQ ID NO:27);
14 (SEQ ID NO:16), 19 (SEQ ID NO:21), 22 (SEQ ID NO:24), 25 (SEQ ID NO:27), and 34 (SEQ ID NO:36);
6 (SEQ ID NO:8), 7 (SEQ ID NO:9), 9 (SEQ ID NO:11), 12 (SEQ ID NO:14), 14 (SEQ ID NO:16), 16 (SEQ ID NO:18), 19 (SEQ ID NO:21), 22 (SEQ ID NO:24), 25 (SEQ ID NO:27), 33 (SEQ ID NO:35), and 34 (SEQ ID NO:36);
6 (SEQ ID NO:8), 9 (SEQ ID NO:11), 16 (SEQ ID NO:18), 19 (SEQ ID NO:21), 22 (SEQ ID NO:24), and 34 (SEQ ID NO:36); and
6(SEQ ID NO:8), 9 (SEQ ID NO:11), 14 (SEQ ID NO:16), 16 (SEQ ID NO:18), 19 (SEQ ID NO:21), 22 (SEQ ID NO:24), 25 (SEQ ID NO:27), and 34 (SEQ ID NO:36).

11. The use of a composition of claim 9 or claim 10 for the manufacture of a medicament for treating sensitivity to *Lol p* V allergen or an allergen which is immunologically cross reactive with *Lol p* V allergen.

12. The use of at least two compositions of claim 9 or claim 10 for the manufacture of a medicament for treating sensitivity to *Lol p* V allergen which is immunologically cross reactive with ryegrass pollen allergen in an individual.

13. The use of a peptide of any one of claims 1 to 5 or 7 for the manufacture of a medicament for treating sensitivity to *Lol p* V allergen or an allergen which is immunologically cross reactive with *Lol p* V allergen.

14. Products containing at least two peptides of any one of claims 1 to 5 or 7 and/or compositions of claim 9 or claim 10 as a combined preparation for simultaneous, separate or sequential use in treating sensitivity to *Lol p* V allergen or an allergen which is cross reactive with ryegrass pollen allergen.

15. An in vitro method of detecting sensitivity to *Lol p* V in an individual, comprising combining a blood sample with at least one peptide of any one of claims 1 to 5 or 7, under conditions appropriate for binding of blood components with the peptide, and determining the extent to which such binding occurs as indicative of sensitivity in the individual to ryegrass pollen; optionally the extent to which binding occurs being determined by assessing B cell function, T cell function, T cell proliferation or a combination of T cell function and B cell proliferation.

16. A therapeutic composition comprising a pharmaceutically acceptable carrier or diluent and at least two *Lol p* V peptides, selected from at least one of the peptides of any one of claims 1 to 5 or 7.

## Patentansprüche

1. Isoliertes Peptidfragment von Lol p V oder ein Homolog davon, bestehend aus einer Aminosäure-Sequenz, gewählt aus den Aminosäure-Sequenzen, wie dargestellt in Figur 2, der Peptide 6 (SEQ ID NO: 8), 7 (SEQ ID NO: 9), 9 (SEQ ID NO: 11), 12 (SEQ ID NO: 14), 16 (SEQ ID NO: 18), 19 (SEQ ID NO: 21), 22 (SEQ ID NO: 24), 28 (SEQ ID NO: 30), 33 (SEQ ID NO: 35), und 34 (SEQ ID NO: 36).

2. Isoliertes Peptidfragment gemäß Anspruch 1, wobei das Peptidfragment mindestens ein B Zeit-Epitop von Lol p V umfasst, gewählt aus der Gruppe, bestehend aus den Aminosäure-Sequenzen, dargestellt in Figur 2, der Peptide 7 (SEQ ID NO: 9), 16 (SEQ ID NO: 18), 19 (SEQ ID NO: 21), 28 (SEQ ID NO: 30) und 34 (SEQ ID NO: 36).

3. Isoliertes Peptidfragment gemäß einem der Ansprüche 1 oder 2, das bei Verabreichung an ein Individuum, das gegenüber einem Lol p V Allergen empfindlich ist, eine T Zell Anergie bei dem Individuum induziert oder das Lymphokin-Sekretions-Profil der T-Zellen bei dem Individuum modifiziert.

4. Isoliertes Peptidfragment gemäß Anspruch 2 oder 3, das entweder
(a) für Lol p V spezifisches Immunglobulin E bei mindestens ungefähr 75% der Individuen, die gegenüber Lol p V empfindlich sind, nicht bindet, oder, wenn eine Bindung des Peptids oder eines Teils davon an das Immunglobulin E auftritt, eine solche Bindung bei einem substantiellen Prozentsatz der gegenüber Lol p V empfindlichen Individuen nicht zu einer Freisetzung von Mediatoren aus Mastzellen oder Basophilen führt; oder
(b) Immunglobulin E in einem geringeren Ausmass als gereinigtes natives Lol p V bindet.

5. Peptidfragment gemäß einem der Ansprüche 1 bis 4, wobei das Peptidfragment durch chemische Synthese erzeugt wird.

6. Isolierte Nukleinsäure, bestehend aus einer Sequenz, die ein Peptid gemäß einem der Ansprüche 1 bis 5 kodiert.

7. Isoliertes Peptidfragment gemäß einem der Ansprüche 1 bis 4, erzeugt in einer Wirtszelle, transformiert mit einer Nukleinsäure gemäß Anspruch 6.

8. Expressionsvektor, kodierend ein Peptidfragment gemäß einem der Ansprüche 1 bis 4.

9. Therapeutische Zusammensetzung, umfassend mindestens ein isoliertes Peptid gemäß einem der Ansprüche 1 bis 5 oder 7 und einen pharmazeutisch akzeptablen Träger oder ein Verdünnungsmittel.

10. Zusammensetzung gemäß Anspruch 9, umfassend eine Kombination von Peptiden, gewählt aus:
14 (SEQ ID NO: 16) und 22 (SEQ ID NO: 24);
19 (SEQ ID NO: 21) und 22 (SEQ ID NO: 24);
19 (SEQ ID NO: 21) und 25 (SEQ ID NO: 27);
14 (SEQ ID NO: 16), 22 (SEQ ID NO: 24) und 25 (SEQ ID NO: 27);
19 (SEQ ID NO: 21), 22 (SEQ ID NO: 24) und 25 (SEQ ID NO: 27);
14 (SEQ ID NO: 16), 22 (SEQ ID NO: 24), 25 (SEQ ID NO: 27) und 34 (SEQ ID NO: 36);
19 (SEQ ID NO: 21 ), 22 (SEQ ID NO: 24), 25 (SEQ ID NO: 27) und 34 (SEQ ID NO: 36);
14 (SEQ ID NO: 16), 19 (SEQ ID NO: 21), 22 (SEQ ID NO: 24) und 25 (SEQ ID NO: 27);
14 (SEQ ID NO: 16), 19 (SEQ ID NO: 21), 22 (SEQ ID NO: 24), 25 (SEQ ID NO: 27) und 34 (SEQ ID NO: 36);
6 (SEQ ID NO: 8), 7 (SEQ ID NO: 9), 9 (SEQ ID NO: 11), 12 (SEQ ID NO: 14), 14 (SEQ ID NO: 16), 16 (SEQ ID NO: 18), 19 (SEQ ID NO: 21), 22 (SEQ ID NO: 24), 25 (SEQ ID NO. 27), 33 (SEQ ID NO: 35) und 34 (SEQ ID NO: 36);
6 (SEQ ID NO: 8), 9 (SEQ ID NO: 11), 16 (SEQ ID NO: 18), 19 (SEQ ID NO: 21), 22 (SEQ ID NO: 24), und 34 (SEQ ID NO: 36); und
6 (SEQ ID NO: 8), 9 (SEQ ID NO: 11), 14 (SEQ ID NO: 16), 16 (SEQ ID NO: 18), 19 (SEQ ID NO: 21), 22 (SEQ ID NO: 24), 25 (SEQ ID NO. 27), und 34 (SEQ ID NO: 36).

11. Verwendung einer Zusammensetzung gemäß Anspruch 9 oder 10 für die Herstellung eines Medikaments für die Behandlung einer Empfindlichkeit auf Lol p V Allergen oder ein Allergen, das immunologisch kreuzreaktiv mit dem Lol p V Allergen ist.

12. Verwendung von mindestens zwei Zusammensetzungen gemäß Anspruch 9 oder 10 für die Herstellung eines Medikaments für die Behandlung einer Empfindlichkeit auf Lol p V Allergen, das immunologisch kreuzreaktiv mit dem Raigras-Pollen-Allergen ist bei einem Individuum.

13. Verwendung eines Peptids gemäß einem der Ansprüche 1 bis 5 oder 7 für die Herstellung eines Medikaments für die Behandlung einer Empfindlichkeit auf Lol p V Allergen oder ein Allergen, das immunologisch mit dem Lol p V Allergen kreuzreaktiv ist.

14. Produkte, enthaltend mindestens zwei Peptide gemäß einem der Ansprüche 1 bis 5 oder 7 und/oder Zusammensetzungen gemäß Anspruch 9 oder 10 als kombinierte Präparation für die simultane, separate oder sequentielle Verwendung bei der Behandlung einer Empfindlichkeit auf Lol p V Allergen oder ein Allergen, das mit dem Raigras-Pollen Allergen kreuzreaktiv ist.

15. In vitro Verfahren für den Nachweis einer Empfindlichkeit auf Lol p V bei einem Individuum, umfassend die Kombination einer Blutprobe mit mindestens einem Peptid gemäß einem der Ansprüche 1 bis 5 oder 7 unter geeigneten Bedingungen für die Bindung von Blutbestandteilen mit dem Peptid und Bestimmung des Ausmasses, in dem eine solche Bindung auftritt als Zeichen der Empfindlichkeit eines Individuums gegenüber Raigras-Pollen; optional wird das Ausmass, mit dem die Bindung auftritt, durch Bewertung der B-Zellfunktion, T-Zellfunktion, T-Zell-Proliferation oder einer Kombination der T-Zellfunktion und B-Zell-Proliferation bestimmt.

16. Therapeutische Zusammensetzung, umfassend einen pharmazeutisch akzeptablen Träger oder ein Verdünnungsmittel und mindestens zwei Lol p V Peptide, gewählt von mindestens einem der Peptide gemäß einem der Ansprüche 1 bis 5 oder 7.

## Revendications

1. Fragment peptidique isolé de Lol pV ou d'un homologue de celui-ci, consistant en une séquence d'acides aminés choisie parmi les séquences d'acides aminés telles que représentées sur la Figure 2 des peptides 6 (SEQ ID N°: 8), 7 (SEQ ID N°: 9), 9 (SEQ ID N°: 11), 12 (SEQ ID N°: 14), 16 (SEQ ID N°: 18), 19 (SEQ ID N°: 21), 22 (SEQ ID N°: 24), 28 (SEQ ID N°: 30), 33 (SEQ ID N°: 35) et 34 (SEQ ID N°: 36).

2. Fragment peptidique isolé selon la revendication 1, dans lequel ledit fragment peptidique comprend au moins un épitope de lymphocyte B de *Lol p*V choisi dans le groupe formé par les séquences d'acides aminés telles que représentées sur la Figure 2 des peptides 7 (SEQ ID N°: 9), 16 (SEQ ID N°: 18), 19 (SEQ ID N°: 21), 28 (SEQ ID N°: 30) et 34 (SEQ ID N°: 36).

3. Fragment peptidique isolé selon la revendication 1 ou 2 qui, lorsqu'il est administré à un individu sensible à l'allergène *Lol p*V, induit une anergie des lymphocytes T chez l'individu ou modifie le profil de sécrétion de lymphokines par les lymphocytes T chez l'individu.

4. Fragment peptidique isolé selon la revendication 2 ou la revendication 3, qui :
(a) ne lie pas l'immunoglobuline E spécifique envers *Lol p*V chez au moins environ 75 % des individus sensibles à *Lol p*V, ou s'il se produit une liaison du peptide ou d'une partie de celui-ci à ladite immunoglobuline E, cette liaison ne conduit pas à la libération de médiateurs par les mastocytes ou les cellules basophiles chez un pourcentage substantiel des individus sensibles à *Lol p*V ; ou bien
(b) lie l'immunoglobuline E à un moindre degré que celui auquel le *Lol p*V naturel purifié lie l'immuno-globuline E.

5. Fragment peptidique selon l'une quelconque des revendications 1 à 4, dans lequel ledit fragment peptidique est produit par synthèse chimique.

6. Acide nucléique isolé consistant en une séquence codant pour un peptide de l'une quelconque des revendications 1 à 5.

7. Fragment peptidique isolé selon les revendications 1 à 4, produit dans une cellule hôte transformée par un acide nucléique de la revendication 6.

8. Vecteur d'expression codant pour un fragment peptidique selon les revendications 1 à 4.

9. Composition thérapeutique comprenant au moins un peptide isolé de l'une quelconque des revendications 1 à 5 ou 7, et un support ou diluant pharmaceutiquement acceptable.

10. Composition selon la revendication 9, comprenant une association de peptides choisie parmi :
14 (SEQ ID N°: 16) et 22 (SEQ ID N°: 24) ;
19 (SEQ ID N°: 21) et 22 (SEQ ID N°: 24) ;
19 (SEQ ID N°: 21) et 25 (SEQ ID N°: 27) ;
14 (SEQ ID N°: 16), 22 (SEQ ID N°: 24) et 25 (SEQ ID N° : 27) ;
19 (SEQ ID N°: 21), 22 (SEQ ID N°: 24) et 25 (SEQ ID N°: 27) ;
14 (SEQ ID N°: 16), 22 (SEQ ID N°: 24), 25 (SEQ ID N°: 27) et 34 (SEQ ID N°: 36) ;
19 (SEQ ID N°: 21), 22 (SEQ ID N°: 24), 25 (SEQ ID N°: 27) et 34 (SEQ ID N°: 36) ;
14 (SEQ ID N°: 16), 19 (SEQ ID N°: 21), 22 (SEQ ID N° : 24) et 25 (SEQ ID N°: 27) ;
14 (SEQ ID N°: 16), 19 (SEQ ID N°: 21), 22 (SEQ ID N°: 24), 25 (SEQ ID N°: 27) et 34 (SEQ ID N°: 36) ;
6 (SEQ ID N°: 8), 7 (SEQ ID N°: 9), 9 (SEQ ID N°: 11), 12 (SEQ ID N°: 14), 14 (SEQ ID N°: 16), 16 (SEQ ID N°: 18), 19 (SEQ ID N°: 21), 22 (SEQ ID N°: 24), 25 (SEQ ID N°: 27), 33 (SEQ ID N°: 35) et 34 (SEQ ID N°: 36) ;
6 (SEQ ID N°: 8), 9 (SEQ ID N°: 11), 16 (SEQ ID N°: 18), 19 (SEQ ID N°: 21), 22 (SEQ ID N°: 24) et 34 (SEQ ID N°: 36) ; et
6 (SEQ ID N°: 8), 9 (SEQ ID N°; 11), 14 (SEQ ID N°: 16), 16 (SEQ ID N°: 18), 19 (SEQ ID N°: 21), 22 (SEQ ID N°: 24), 25 (SEQ ID N°: 27) et 34 (SEQ ID N°: 36).

11. Utilisation d'une composition de la revendication 9 ou la revendication 10 pour la fabrication d'un médicament destiné au traitement d'une sensibilité à l'allergène *Lol p*V ou à un allergène qui présente une réactivité immunologique croisée avec l'allergène *Lol p*V.

12. Utilisation d'au moins deux compositions de la revendication 9 ou la revendication 10 pour la fabrication d'un médicament destiné au traitement d'une sensibilité à l'allergène *Lol p*V qui présente une réactivité immunologique croisée avec un allergène du pollen de ray-grass chez un individu.

13. Utilisation d'un peptide de l'une quelconque des revendications 1 à 5 ou 7 pour la fabrication d'un médicament destiné au traitement d'une sensibilité à l'allergène *Lol p*V ou à un allergène qui présente une réactivité immunologique croisée avec l'allergène *Lol p*V.

14. Produits contenant au moins deux peptides de l'une quelconque des revendications 1 à 5 ou 7 et/ou compositions de la revendication 9 ou la revendication 10, sous forme d'une préparation combinée pour une utilisation simultanée, séparée ou séquentielle dans le traitement d'une sensibilité à l'allergène *Lol p*V ou à un allergène qui présente une réactivité croisée avec un allergène du pollen de ray-grass.

15. Méthode *in vitro* de détection de la sensibilité à *Lol p*V chez un individu, comprenant les étapes consistant à combiner un échantillon de sang avec au moins un peptide de l'une quelconque des revendications 1 à 5 ou 7, dans des conditions appropriées pour la liaison de composants du sang avec le peptide, et à déterminer le degré auquel cette liaison se produit comme indication de la sensibilité chez l'individu au pollen de ray-grass ; le degré auquel la liaison se produit étant facultativement déterminé en évaluant la fonction des lymphocytes B, la fonction des lymphocytes T, la prolifération des lymphocytes T ou une combinaison de la fonction des lymphocytes T et de la prolifération des lymphocytes B.

16. Composition thérapeutique comprenant un support ou diluant pharmaceutiquement acceptable et au moins deux peptides de *Lol p*V choisis parmi au moins l'un des peptides de l'une quelconque des revendications 1 à 5 ou 7.
